# EUROPEAN PATENT SPECIFICATION

(11) **EP 4 409 293 B1**
(45) Date of publication and mention of the grant of the patent: **07.10.2026**
(21) Application number: 22797313.8
(22) Date of filing: 26.09.2022
(51) Int. Cl.: G01N 33/58, G01N 33/68, G01N 33/92

(54) **QUANTIFICATION METHOD OF POLYSORBATE IN AQUEOUS FORMULATIONS**
VERFAHREN ZUR QUANTIFIZIERUNG VON POLYSORBAT IN WÄSSRIGEN FORMULIERUNGEN
PROCÉDÉ DE QUANTIFICATION DE POLYSORBATE DANS DES FORMULATIONS AQUEUSES

(30) Priority: 27.09.2021 EP 21199048
(43) Date of publication of application: 07.08.2024
(60) Divisional application: 26202634.7
(73) Proprietor: Boehringer Ingelheim International GmbH, 55216 Ingelheim am Rhein (DE)
(72) Inventor: HAEMMERLING, Frank, 55216 Ingelheim am Rhein (DE); BAUMGARTNER, Kai, 55216 INGELHEIM AM RHEIN (DE); BUSKE, Julia, 55216 Ingelheim am Rhein (DE); GARIDEL, Patrick, 55216 Ingelheim am Rhein (DE); MOLNAR, Daniel, 55216 Ingelheim am Rhein (DE)
(74) Representative: Lutze, Oliver
(86) International application number: PCT/EP2022/076661
(87) International publication number: WO 2023/046953

(56) References cited:
- MARTOS ARIADNA ET AL: "Novel High-Throughput Assay for Polysorbate Quantification in Biopharmaceutical Products by Using the Fluorescent Dye DiI", vol. 109, no. 1, 10 October 2019 (2019-10-10), US, pages 646 - 655, XP055830742, ISSN: 0022-3549, Retrieved from the Internet <URL:https://www.jpharmsci.org/article/S0022-3549(19)30648-3/pdf> DOI: 10.1016/j.xphs.2019.10.013
- LIPPOLD STEFFEN ET AL: "Impact of mono- and poly-ester fractions on polysorbate quantitation using mixed-mode HPLC-CAD/ELSD and the fluorescence micelle assay", JOURNAL OF PHARMACEUTICAL AND BIOMEDICAL ANALYSIS, ELSEVIER B.V, AMSTERDAM, NL, vol. 132, 26 September 2016 (2016-09-26), pages 24 - 34, XP029801517, ISSN: 0731-7085, DOI: 10.1016/J.JPBA.2016.09.033

## Description

### BACKGROUND OF THE INVENTION

### TECHNICAL FIELD

The invention relates to a quantification method of polysorbate in aqueous formulations in the presence of one or more polypeptides.

### STATE OF THE ART

Non-ionic surfactants emerged as excipients of choice in aqueous biopharmaceutic formulations to stabilize the protein against interfacial stresses, to saturate aggregation prone areas of the protein or help to prevent adsorption. Thereby, they help to maintain the colloidal and conformational stability of the active pharmaceutical ingredient (API), and hence, the biotherapeutic activity and efficacy. The most widely used surfactants for aqueous biopharmaceutical formulations are polysorbates (PS), also known as Tween^{®}, due to their biocompatibility and low toxicity [1]. But polysorbates exhibit a number of degradation reactions, in particular oxidation and (enzymatic) hydrolysis, whereby numerous degradation products are formed and functional properties are lost, potentially leading to the instability of the aqueous formulation. Therefore, the degradation of polysorbate might lead to particle formation in aqueous formulations, which can be a major quality concern and potential risk factor [2].

Due to the known degradation of the polysorbates, for example, used to stabilize biopharmaceutical formulations or biotherapeutic products, the presence and the concentration of polysorbates should be closely monitored, i.e. during the development, manufacturing, and also along the shelf-life and storage time of aqueous formulations.

Known methods for determining the polysorbate content are, for example, based on mass spectrometry, evaporative light scattering detection, charged aerosol detection, nuclear magnetic resonance, and also combined methods such as liquid chromatography with charged aerosol detection (LC-CAD) or LC-evaporative light scattering detection with a mixed mode on-line solid phase extraction (SPE) cartridge and an acidified mobile phase. These methods require expensive equipment, are time-consuming, personnel-intensive and sometimes require complex sample preparation. In addition, most of the methods are not compatible with the presence of proteins and require a protein removal step.

Therefore, a number of different assays for the quantitative analysis of polysorbates in biopharmaceutical products are known, however, HPLC techniques in combination with charged aerosol detection (CAD) and HPLC techniques in combination with the fluorescence micelle assay (FMA) belong to the most extensively used techniques for the quantification of polysorbates [3].

The fluorescence micelle assay (FMA) is based on the partitioning of the hydrophobic dye N-phenyl-1-naphthylamine (NPN) into surfactant micelles. NPN exhibits a low-fluorescence signal in aqueous environments, which increases in more hydrophobic environments such as the core of the micelles. Polysorbates which are non-ionic surfactants form such micelles in aqueous solution. The micelles are formed at and above the critical micelle concentration (CMC), which represents the concentration of the surfactant at and above which the micelles being formed. For polysorbates the CMC at 25 °C ranges between 15 - 75 µM for polysorbate 20 (PS20 HP) and is considerably lower for polysorbate 80 (PS80 HP) with 7 - 16 µM [4]. Therefore, the fluorescence micelle assay is applied for the determination of the polysorbate content in samples with a polysorbate concentration at and above the critical micelle concentration (CMC). In fact, the CMC of polysorbates is significantly lower than the concentration of interest in aqueous formulations to be determined.

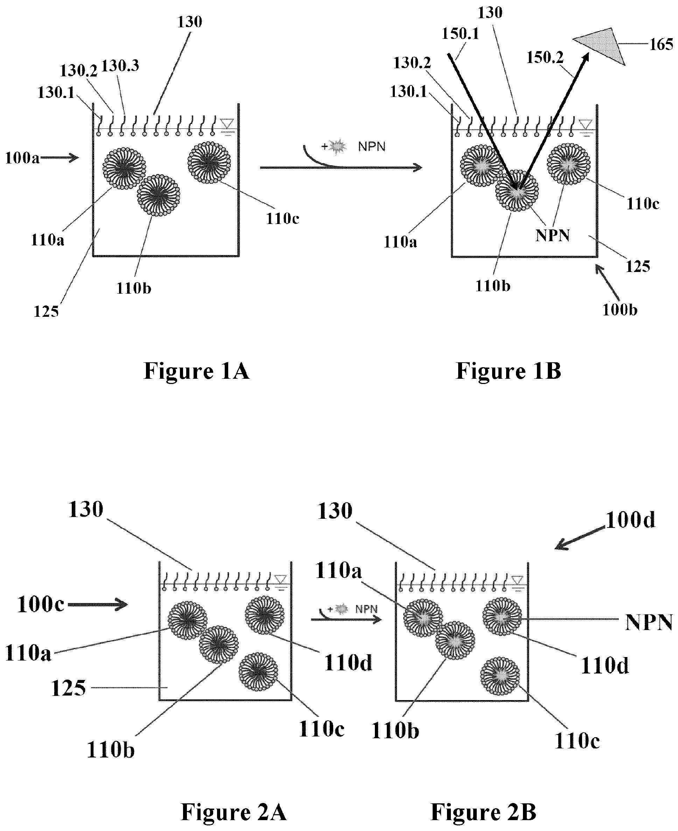

For a better understanding the principle of the fluorescence micelle assay is explained in the Figures 1 to 3 as follows:
In Figures 1A and 1B the concept of the fluorescence micelle assay is shown in schematic illustrations. In Fig. 1A a polysorbate containing sample 100a having water 125 as solvent is depicted whereby a number of polysorbate molecules 130, namely 130.1, 130.2, 130.3, ... are present at the air-liquid interface. The hydrophobic tails of the polysorbate molecules 130 extend out of the water surface whereas the hydrophilic head is directed into the water. In the sample 100a the concentration c of the polysorbate (PS) is selected to be equal or higher than the critical micelle concentration (c(PS) ≥ CMC), i.e., the polysorbate forms micelles. In the aqueous solvent 125 three micelles 110a, 110b and 110c are schematically illustrated each of which has a spherical shape. In each micelle 110a, 110b, or 110c, respectively, the hydrophilic head of each of the polysorbate molecules 130 is located on the outside of the sphere and the hydrophobic tail of each of the polysorbate molecules 130 is directed to the inside of the sphere, respectively.

If the hydrophobic dye N-phenyl-1-naphthylamine (NPN) is added to the sample 100a the dye is "incorporated" into the nonpolar core of polysorbate micelles 110a, 110b, and 110c (schematically shown by an NPN symbol in the middle of each sphere) as schematically shown in Fig. 1B. Due to the partitioning of the NPN dye into the nonpolar core of the micelles, it results a significantly increased fluorescence signal.

In Fig. 1B the irradiated light is schematically illustrated by arrow 150.1 and the emitted fluorescence light is schematically illustrated by arrow 150.2 which is detected in a fluorescence detector 165. This characteristic allows the determination of the micelle concentration, and therefore the polysorbate concentration, by measuring the fluorescence signal of the N-phenyl-1-naphthylamine (NPN) present in the micelles. This method was firstly described by Brito et al. [5] where the N-phenyl-1-naphthylamine was used for the determination of the critical micelle concentration (CMC) of different surfactants.

In Figures 2A and 2B the same embodiment as in Figs. 1A and 1B is shown whereby the polysorbate is polysorbate 20 (PS20). The polysorbate 20 has formed micelles 110a, 110b, 110c and 110d which are completely intact. In this embodiment the fluorescence signal obtained by fluorescence micelle assay is a very strong signal.

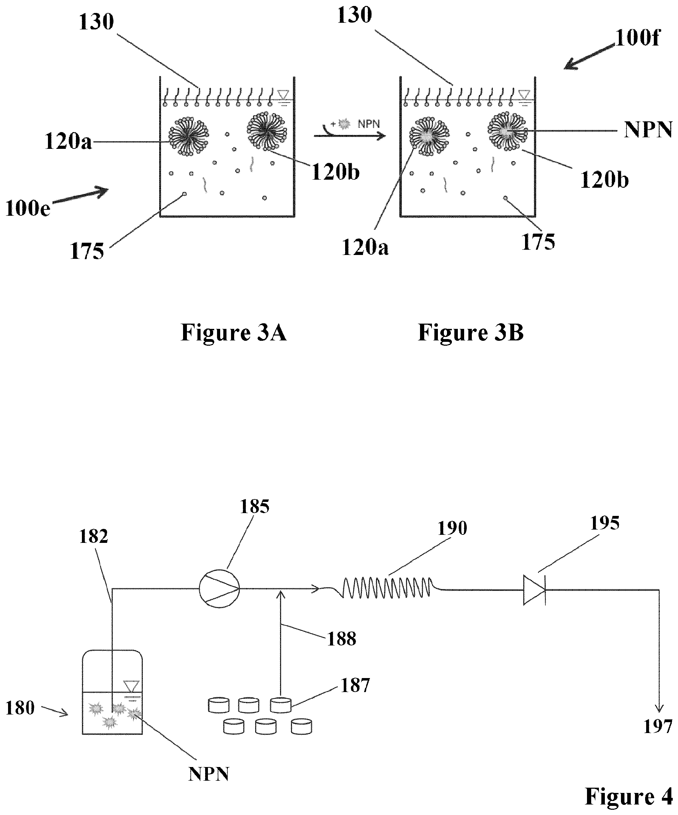

In Figures 3A and 3B the polysorbate is polysorbate 20 (PS20) which has been partially degraded. As may be seen the micelles 120a and 120b may change their properties such as a destabilization of the initial micelles or reduce the micelle concentration. Degradation products 175 from the intact polysorbate from the micelles 120a and 120b of the polysorbate 20 are formed, and depending on their physicochemical properties such as increased polarity, are now present in the aqueous phase. In this embodiment the fluorescence signal obtained by fluorescence micelle assay is a decreased signal.

As a result, the fluorescence micelle assay may be used as a method to determine the content of polysorbates in aqueous formulations, whereby fluorescence micelle assays are simple to perform and allow a rapid determination of the polysorbate content.

One widely applied method to determine the concentration of polysorbate is the fluorescence micelle assay (FMA) conducted on a high-performance liquid chromatography (HPLC) system. In such a case, the fluorescence micelle assay is performed using a HPLC system which is connected to a fluorescence detector as direct flow analysis of the fluorescent dye present in the mobile phase.

Figure 4 shows a schematically simplified diagram of a fluorescence micelle assay (FMA) conducted on a HPLC system. The mobile phase 180 comprises the fluorescent dye in form of N-phenyl-1-naphthylamine (NPN), an organic solvent such as acetonitrile, a buffer such as tris(hydroxymethyl)aminomethan (Trizma base) or tris(hydroxymethyl)methylamin, and optionally appropriate additives, for example, a salt such as NaCl, and a tenside, e.g. a non-ionic tenside such as polyalkylenglycolether (known brands are Brij^{®}, Genapol^{®}, Lutensol^{®}). The mobile phase 180 is taken through the capillary tubing 182 controlled by the pump 185 having a suitable flow rate. The sample 187 containig the polysorbate in aqueous solution is removed through capillary tubing 188 and combined with the mobile phase 180. The polysorbate containing mobile phase 180 is then guided through a reaction loop 190 and after that conveyed to the fluorescence detector 195 where the fluorescence signal is measured. After the sample containing polysorbate has been analysed, it can be disposed as waste 197. For quantification, the polysorbate peak area is determined using a calibration curve [3, 6]. As most HPLC devices are equipped with an autosampler, the fluorescence micelle assay can also be performed automated on HPLC systems.

Although this approach of the determination of the polysorbate concentration appears to be a direct straightforward method, the fluorescence micelle assay performed on a HPLC system comes along with a number of drawbacks, such as an analysis time of approximately two minutes per sample which constitutes an analytical bottleneck, especially for huge sample sets. Furthermore, the non-specific adsorption of the fluorescent dye N-phenyl-1-naphthylamine (NPN) to all accessible surfaces, especially of the capillaries and valves of the chromatographic system, requires long flushing times with dye containing mobile phase to saturate all surfaces with NPN. This adsorption of NPN causes the need of a specially adapted and permanently assigned HPLC-system for performing the fluorescence micelle assay. In addition to that, also desorption of the dye NPN might lead to a falsification of the surfactant concentration measurement.

In addition, it is well-known in prior art that the fluorescence micelle assay is not suitable to be used in the presence of protein(s). The hydrophobic fluorescence dye NPN shall interact with other hydrophobic components within the biotherapeutic formulations such as proteins. The known auto- or self-fluorescence of components within the biotherapeutic formulations, for example proteins, could excite NPN (350 nm) which might lead to errors in the quantification [7, 12]. The up-to-now prior art agreed on the finding that the fluorescence micelle assay is not possible in the presence of a protein [3, 6, 8, 9, 10, 11, 12]. An additional prior art search has confirmed that the fluorescence micelle assay has not successfully being performed in the presence of a protein up to now.

Still in 2020 the publication [9] comes to the result that the fluorescence micelle assay failed to quantify polysorbate 20 in the presence of protein (see p. 653/4, bridging paragraph). Thus, up-to-date publications about the fluorescence micelle assay report data only in the absence of protein. As a result, a preparative protein-removal step is considered to be required in order to perform the fluorescence micelle assay for aqueous formulations.

Literature [3] also points out that the N-phenyl-1-naphthylamine (NPN) reaction shall not react uniformly for the different polysorbate-esterified species, which should lead to a misestimation of the polysorbate content.

However, the present invention has been developed against an opinion or preconceived idea widely or universally held by experts in that technical field according to which fluorescence micelle assay in the presence of a protein shall not be feasible.

It is therefore an object of the present invention to avoid the deficiencies of prior art and to provide a method for the quantification of polysorbate which overcomes the drawbacks of fluorescence micelle assay performed on a HPLC-system.

Furthermore, it is a further object to provide a method for the quantification of polysorbate which may be also suitable to perform high-throughput analysis of polysorbate containing samples.

### SUMMARY OF THE INVENTION

Surprisingly, it was found that the disadvantages known from prior art may be overcome by performing a fluorescence micelle assay (FMA) using N-phenyl-1-naphthylamine (NPN) as fluorescence dye without a HPLC-system wherein polysorbate contained in a liquid sample is quantifiable in the presence of one or more peptides or polypeptides as well as proteins as described in the appended claims.

Therefore, it is provided a method for the quantification of polysorbate in an aqueous formulation containing polysorbate and one or more polypeptides having the following steps:
(i) providing a number of samples of the aqueous formulation;
(ii) adding of a solution containing the fluorescent dye N-phenyl-1-naphthylamine to each of the samples, respectively, to obtain fluorescent dye containing samples to be tested in a fluorescence micelle assay;
(ii') optionally incubating the fluorescent dye containing samples of step (ii);
(iii) performing a fluorescence micelle assay with the fluorescent dye containing samples obtained in step (ii) or (ii') and
(iv) determining the content of polysorbate present in each of the samples from the fluorescence micelle assay performed in step (iii);
with the proviso that the method is being performed without the use of a HPLC-system,
characterized in that
the method steps (i) to (iv) are performed on a microplate.

It was unexpected that any kind of polypeptide, particularly protein, present in the starting aqueous formulation does not interfere with the measurement of the fluorescence micelle assay. Furthermore, it was also unexpected that impurities, additives, excipients, do not interfere with the measurement of the fluorescence micelle assay.

Indeed, the methods of the present invention were found to reliably provide the content of polysorbate in aqueous formulations wherein at the same time one or more peptides, polypeptides, or proteins are present. In addition, the quantification can be employed for a series of test sets at the same time for a high-throughput quantitative analysis of polysorbate in samples containing peptide(s), polypeptide(s), or protein(s).

The invention is also directed to the use of the method according to the invention for the quantification of polysorbate in an aqueous formulation, for example, as a stability test of the aqueous formulation, during the development, manufacture, shelf life or storage time thereof, whereby the aqueous formulation contains polysorbate and one or more (poly)peptides as disclosed herein, with the proviso that the method is being performed without the use of a HPLC-system and the fluorescence micelle assay is performed in the presence of one or more polypeptides, including one or more proteins.

### BRIEF DESCRIPTION OF THE DRAWINGS

Embodiments of the prior art and of the present invention will be described by way of example with reference to the accompanying figures, which are schematic and are not intended to be drawn to scale so that no assumption of precise geometric values can be made regarding the original size. The figures of the present disclosure are incorporated in and constitute a part of the specification, also illustrating embodiments of the invention without limitation to the specific embodiments described. The drawings together with the general description and detailed description serve to explain the principles of the present disclosure. The same features are denoted by the same reference signs throughout the figures. It is shown:
- Figures 1A and 1B: a schematic illustration of the concept of the fluorescence micelle assay;
- Figures 2A and 2B: a schematic illustration of a sample containing polysorbate 20 (PS20) before and after adding the dye N-phenyl-1-naphthylamine used in the fluorescence micelle assay, whereby polysorbate 20 forms intact micelles;
- Figures 3A and 3B: a schematic illustration of a sample containing polysorbate 20 (PS20) before and after adding the dye N-phenyl-1-naphthylamine used in the fluorescence micelle assay, whereby polysorbate 20 has been partially degraded and forms no or not intact micelles;
- Figure 4: a schematically simplified diagram of a fluorescence micelle assay (FMA) conducted on a HPLC system;
- Figure 5: a schematic illustration according to an embodiment of the present invention;
- Figure 5A: a schematic illustration of an embodiment of a microplate;
- Figure 6: a linearity plot of the fluorescence signal in a fluorescence micelle assay according to an embodiment of the present invention with increasing polysorbate concentrations;
- Figure 7A to 7F: diagrams showing the determination of polysorbate concentrations for the investigation of the specificity of the fluorescence micelle assay according to an embodiment of the present invention with or without polysorbate in the presence or absence of different (poly)peptides.

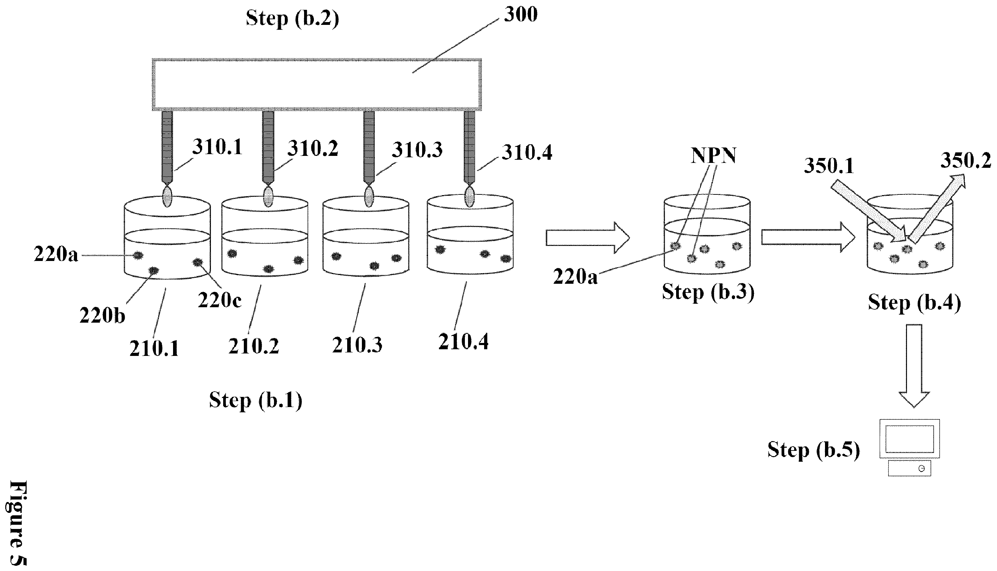

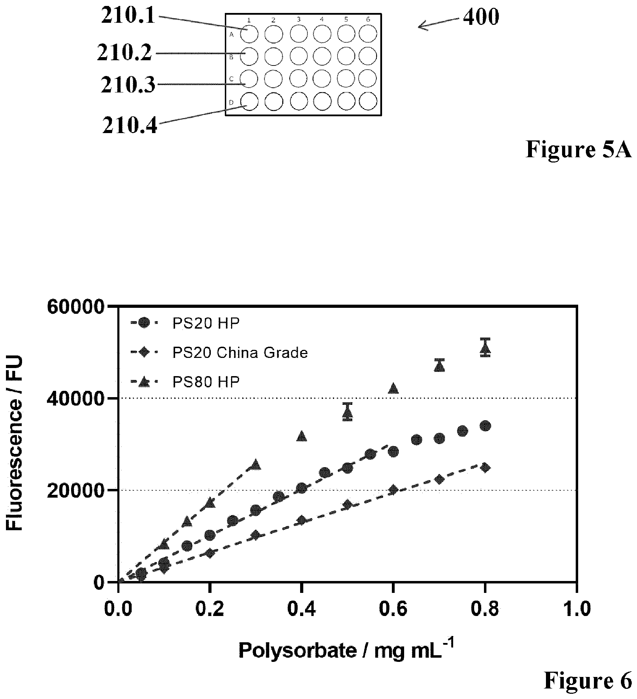

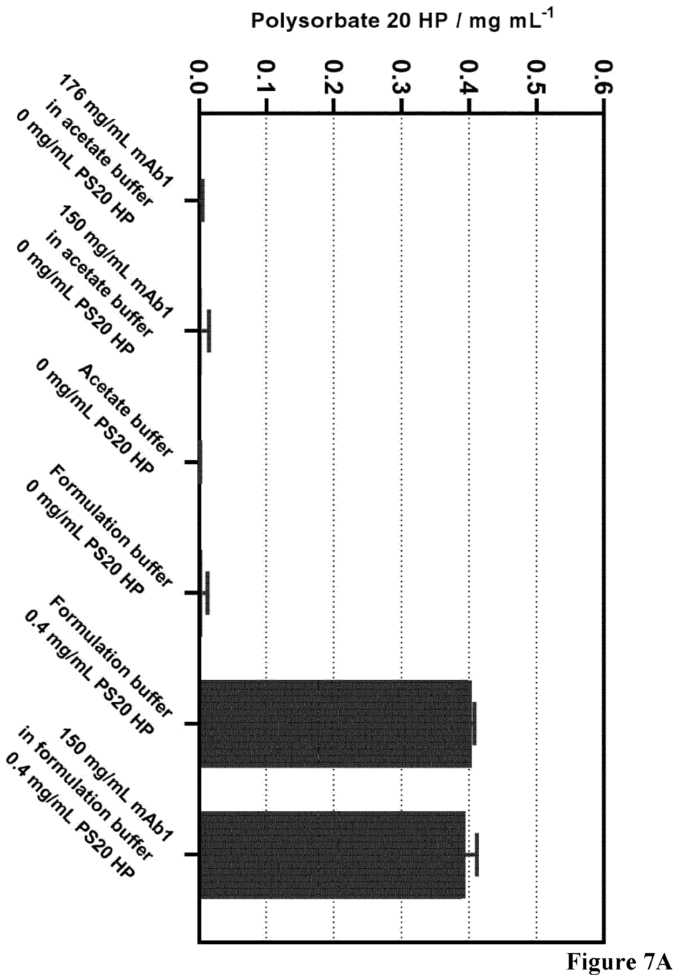

Figures 1A, 1B, 2A, 2B, 3A, 3B, and 4 have been already described in view of several embodiments of the prior art. Figure 5 will be described later in the context of an embodiment of the present invention. Figures 6 and 7A to 7F will be explained in the experimental section.

### DETAILED DESCRIPTION

### DEFINITION OF GENERAL TERMS

Terms not specifically defined herein should be given the meanings that would be given to them by a person skilled in the art in light of the disclosure and the context.

The expression "quantification of polysorbate" shall be understood in its broadest sense and stands for the determining of the concentration or amount of polysorbate present in an aqueous formulation from which a sample is taken and examined. The quantification is achieved by using a fluorescence micelle assay (FMA).

A "fluorescence micelle assay (FMA)" is a testing method known by the person skilled in the art, for which a variety of literature is available [3, 5, 9] so that the skilled person knows the details of this procedure and is familiar with it, for example, the sample amount which may be used, the appropriate solvents to be used, the characteristics of the fluorescent dye N-phenyl-1-naphthylamine such as the light sensitivity to be taken into account, the composition of the solution of the fluorescent dye added to a sample etc.

The expression "polysorbate" or "polysorbates" which are used interchangeably and synonymously represent a class of emulsifiers or non-ionic surfactants used in pharmaceuticals, cosmetics and food. They are often used to solubilise products in water that are not water-soluble. Polysorbates are oily liquids derived from ethoxylated sorbitan (a derivative of sorbitol) esterified with fatty acids. Well-known brand names are Kolliphor, Scattics, Alkest, Canarcel and Tween. Commercially available polysorbates usually consist of a mixture of structurally related molecules such as nonesterified sorbitan and/or isosorbitan-polyethylene glycol (PEG) species, mono-, di-, tri-, tetra-esters, fatty acids, and the like.

Exemplary representatives of polysorbates are
polysorbate 20 (PS20) or polyoxyethylene (20) sorbitan monolaurate;
polysorbate 40 (PS40) or polyoxyethylene (20) sorbitan monopalmitate;
polysorbate 60 (PS60) or polyoxyethylene (20) sorbitan monostearate; and
polysorbate 80 (PS80) or polyoxyethylene (20) sorbitan monooleate.

The numbers 20, 40, 60 and 80 which follow the term "polysorbate" stand for the main type of fatty acid associated with the polyoxyethylene sorbitan residue of the molecule, i.e. monolaurate is indicated by 20, monopalmitate is indicated by 40, monostearate is indicated by 60, and monooleate is indicated by 80.

The number 20 following the term "polyoxyethylene" refers to the total number of oxyethylene -(CH₂CH₂O)- groups present in the molecule.

In the present invention the main focus lies on polysorbate 20 and 80 which are mostly used in the pharmaceutical industry, whereas polysorbate 40 and 60 are more commonly used in the food or cosmetics industry.

According to the European Pharmacopoeia (Ph. Eur.) specification, for the content of fatty acids, the laurate ester accounts for 40-60 % of esters in polysorbate 20, the remaining esters range from C8 to C18. The oleate ester accounts for 58-85 % of esters in polysorbate 80, the remainder range from C14 to C18 including stearate, linoleate, and linoleneate esters.

The term "aqueous" is intended to mean that water is present in the formulations. For example, water can constitute a part of the solvent, e.g. a small part or the main part of the solvent or can represent the only solvent present. In fact, any type of water may be used. Purified water may be preferred but according to some embodiments also tap water may be used. The type of water selected depends from the intended use of the aqueous formulation. Purified water used according to the present invention is water that has been undergone a purification process such as distillation, reverse osmosis, carbon filtering, capacitive or electro-deionization, micro- or ultrafiltration, ultraviolet oxidation or the like to remove impurities to be suitable for use. Combinations of these processes may also be used in order to achieve water of such high purity, e.g. ultrapure water, that its trace contaminants are measured in parts per billion (ppb) or parts per trillion (ppt). In one embodiment, water used in the method of the invention is ultrapure water, for example ultrapure water of type 1 (Milli-Q^{®} water) according to ASTM D1193 or ISO 3696. According to another embodiment, the water used may be sterile water suitable for administration to a subject such as water for injection (WFI). Also, distilled water, bidistilled or deionized water may be used.

The term "aqueous formulation" refers to a solution in which the solvent or one of the solvents is water. The solution comprises a true solution, dispersion, suspension and the like, unless otherwise stated. The aqueous formulation according to the invention comprises polysorbate and one or more (poly)peptides and optionally excipient(s).

The term "sample" is understood as broadly as possible to mean a limited or smaller quantity of the aqueous formulation, which is taken for analysis. The sampling may be done manually or by an automated method.

The expressions "(poly)peptide" or "(poly)peptides" which are summarized in the term "(poly)peptide(s)" comprise "peptide", "peptides", "polypeptide", "polypeptides", "proteins" and "protein", whereby "protein" or "proteins" are usually considered to be comprised by "peptide(s)" or "polypeptide(s)". However, for better understanding and to ensure that the term polypeptide(s) also includes proteins, the proteins are listed in addition to the peptides and polypeptides herein. That is, "polypeptides" or "one or more polypeptides" stand for one or more polypeptides, which includes one or more proteins. Therefore, peptide(s), polypeptide(s) and protein(s) and variations of these terms refer to peptide(s), oligomer(s) such as oligopeptide(s), polypeptide(s) or protein(s) including fusion protein(s), respectively. As generally known a peptide is an organic compound containing peptide bonds between amino acids. According to their number, oligopeptides with few amino acids are distinguished from polypeptides with many amino acids. For example, a peptide may comprise at least two amino acids joined to each other for example by a normal peptide bond, or, alternatively, by a modified peptide bond, such as in the cases of isosteric peptides. For simplification, the terms "peptide", "polypeptide", or "protein" can be interchangeably used in the present application, the skilled person is familiar with the terms per se and understands their meaning. A peptide, a polypeptide, or a protein can be composed of L-amino acids and/or D-amino acids. Preferably, a peptide, polypeptide, or protein is either (entirely) composed of L-amino acids or (entirely) of D-amino acids, thereby forming "retro-inverso peptide sequences".

The terms "peptide", "polypeptide", or "protein" particularly refers to a "classical" peptide, polypeptide or protein, whereby a "classical" peptide, polypeptide or protein is typically composed of amino acids selected from the 20 amino acids defined by the genetic code, linked to each other by a peptide bond. The terms "peptide", "polypeptide" or "protein" include, in addition to these amino acids, amino acids other than the 20 defined by the genetic code, or they may be composed of amino acids other than the 20 defined by the genetic code. In particular, a peptide, polypeptide or protein in the context of the present invention can equally be composed of amino acids modified by natural processes, such as post-translational maturation processes or by chemical processes, which are well known to a person skilled in the art.

The terms "peptide", "polypeptide", "protein" also include modified peptides, polypeptides and proteins. For example, peptide, polypeptide, or protein modifications can include acetylation, acylation, ADP-ribosylation, amidation, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bond formation, demethylation, glycosylation including pegylation, hydroxylation, iodization, methylation, myristoylation, oxidation, proteolytic processes, phosphorylation, prenylation, racemization, seneloylation, sulfatation, amino acid addition such as arginylation or ubiquitination. In particular, the terms "peptide", "polypeptide", or "protein" also include "peptidomimetics" which are defined as peptide analogs containing non-peptidic structural elements, which peptides are capable of mimicking or antagonizing the biological action(s) of a natural parent peptide.

The peptide, polypeptide, or protein may be a therapeutic peptide, therapeutic polypeptide, or therapeutic protein, which are used for the prevention or treatment of any disease or any disorder. The (poly)peptide, particularly peptide, polypeptide or protein, may be an "antigen-binding molecule" which is capable of binding to a target antigen, and encompasses monoclonal antibody, polyclonal antibody, monospecific antibody, multispecific antibody (e.g., bispecific antibody), single chain antibodies or and antibody fragments (e.g. Fv, scFv, Fab, Fab', scFab, F(ab')2, Fab2, Fc and Fc'-fragments), heavy and light immunoglobulin chains and their constant, variable or hypervariable region as well as Fv- and Fd-fragments, diabodies, triabodies, scFv-Fc, minibodies, single domain antibodies (e.g. VhH), etc.), as long as they display binding to the relevant target molecule(s) or a mixture thereof. According to the invention, in addition to proteins in the form of one or more antibodies, proteins may also be present in the form of one or more enzymes. Sizes of enzymes range from just 62 amino acid residues, for the monomer of 4-oxalocrotonate tautomerase (approx. 6.8 kDa to over 2,500 residues (approx. 275 kDa) in the animal fatty acid synthase. Enzymes that belong to the protein group are included here, such as peptidases, glycosidases, lipases, nucleases and lactase, but not ribozymes, which are only made up of RNA.

In addition, it should be noted that in this disclosure, the singular and plural forms are not used in a restrictive manner. As used herein, the singular forms "a", "an", "one" and "the" therefore refer to both the singular and the plural, unless otherwise indicated or apparent from the context.

The expressions "comprising", "comprise", "comprised", "containing", "contain" or "contained" shall also encompass the more specific term "consisting of" unless otherwise stated or apparent from the context.

The expression "about" or "approximately" means within 20 %, particularly within 10 % and more particularly within 5 % of a given value or upper or lower range value.

### DETAILED DESCRIPTION

In the following multi-step methods are described. Optimum method conditions and parameters for each individual step may vary depending on the particular aqueous formulation, the optional additive(s), optional excipient(s), solvent(s) beside water selected and (poly)peptide(s), present. Unless otherwise specified, the method conditions and parameters of each method step may be readily selected by one of ordinary skill in the art based on the present disclosure. Exemplary procedures are provided in the experimental section.

A non-claimed method (a) has been developed to quantify polysorbates in aqueous formulations containing polysorbate and one or more (poly)peptides:
In a first step (a.1) a sample of an aqueous formulation containing polysorbate and one or more (poly)peptides is provided.

The polysorbate may be a polysorbate which is selected from polysorbate 20, polysorbate 40, polysorbate 60, or polysorbate 80, particularly polysorbate 20 or polysorbate 80.

The polysorbate may be intended for pharmaceutical use, particularly for use in drugs. Particularly, the polysorbate 20 or polysorbate 80 is pharmaceutically acceptable and may have pharma grade quality such as Ph. Eur./USP/China Pharmacopoeia grade quality. For example, the polysorbate may be selected from polysorbate 20 or polysorbate 80 which are suitable for parenteral administration.

The polysorbate 20 may be selected from the following commercial products: Super Refined^{™}PS20, Tween^{™}20, Tween^{™}20 HP, Tween^{™} 20 pharma grade or PS20 HP, as available from Croda Europe Ltd., or PS20 China grade, as available from Nanjing Well Pharmaceutical Co., Ltd. In view of polysorbate 80 this may be selected from the following commercial products: Super Refined^{™}PS80, Tween^{™}80, Tween^{™}80 HP, Tween^{™} 80 pharma grade or PS80 HP, as available from Croda Europe Ltd, or PS80 China grade, as available from Nanjing Well Pharmaceutical Co., Ltd.

Any peptide, polypeptide, or protein known to the person skilled in the art can be used as already explained, regardless of its type, characteristics and size. Also a mixture of two or more (poly)peptides such as peptides, polypetides, proteins may be present in the aqueous formulation.

The sampling can be carried out in any procedure known to the expert, for example manually by a person or by an automated device or system. It is a matter of course that the sampling should be carried out in such a way that the following analytical procedure is not adversely affected or compromised. The amount of the sample withdrawn from the aqueous formulation wherein the polysorbate content is to be quantified depends on several factors such as the type and composition of the aqueous formulation, the type and size of the sample container into which the sample(s) is(are) filled, the type and size of the fluorescence micelle assay test device etc. It is also possible that the volume of the sample may be adjusted prior to perform the fluorescence micelle assay. For example, the sample may be diluted, e.g. with water as solvent, or the sample may be concentrated. The change of the sample volume must then be taken into account when quantifying the polysorbate in the starting aqueous formulation later on.

In the following step (a.2) the solution containing the fluorescent dye N-phenyl-1-naphthylamine is added to the sample obtained in step (a.1) in order to obtain a fluorescent dye containing sample to be tested in a subsequent fluorescence micelle assay.

The fluorescence micelle assay method is based on the detection of the fluorescence emission intensity of the fluorescent dye N-phenyl-1-naphthylamine in the presence of polysorbate at concentrations at or above their critical micelle concentration. N-phenyl-1-naphthylamine (C₁₆H₁₃N) has the following chemical structure 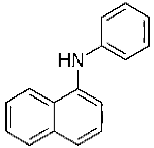 and is commercially available. Since it is hardly soluble in water a solution containing an organic solvent is prepared or provided. The solution containing the dye in step (a.2) usually has the same components and in particularly also the same composition, as used for the mobile phase in a combined FMA-HPLC method in the prior art (see, for example, literature [9], p. 649, 3rd paragraph: "The mobile phase contained 150 mM sodium chloride, 50 mM tris(hydroxymethyl) methylamin, pH 8.0, 5 % (v/v) ACN, 15 ppm Brij35, and 5 mM of NPN.").

Such a solution containing the fluorescent dye in step (a.2) may usually contain the hydrophobic dye in the range from 1 µM to 10 µM, particularly 5 µM, an organic solvent such as acetonitrile in the range from1 %(v/v) to 10 %(v/v), particularly 5 %(v/v), a buffer such as tris(hydroxymethyl)aminomethan (Trizma base) or tris(hydroxymethyl)methylamin in the range from 5 mM to 500 mM, particularly 50 mM, a salt such as NaCl in the range from 10 mM to 1000 mM, particularly 150 mM, and a tenside, e.g. a non-ionic tenside such as polyalkylenglycolether (known brands are Brij^{®}, Genapol^{®}, Lutensol^{®}) in the range from 0.00015 % to 0.015 %, particularly 0.0015 %. The solution is typically a buffer solution wherein the components are present to solubilize the fluorescent dye. Other compositions of the dye containing solution are also possible in so far as they allow to conduct the subsequent fluorescence micelle assay.

It can be advantageous if the solution comprising the fluorescent dye N-phenyl-1-naphthylamine is mixed prior to adding it to the sample in step (a.2). The mixing may be conducted by shaking or stirring, for example. A shaking may be employed, for example, by using an orbital shaker in order to achieve a shaking which is especially effective in a very short time.

Such a mixing of the solution may remove any concentration gradient of the dye due to unspecific adsorption on the surface of the reservoir and may contribute to have a more homogeneous solution of the dye.

Method step (a.3) is an optional method step. In individual cases, however, it can be advantageous if incubation is carried out because the partitioning of the fluorescence dye into the inner core of the polysorbate micelles could be improved. Thus, according to optional method step (a.3) the sample obtained in step (a.2) is incubated.

The incubating may be performed under shaking.

The incubating temperature may be selected from the range of from 10 to 60 °C, particularly 30 to 40 °C, more particularly 35 °C. The incubation temperature may be readily selected from the above disclosed range.

The incubation time may be selected according to the knowledge of the average skilled person. Also no or shorter or longer incubation times are possible; the skilled person is readily able to select a suitable incubation temperature and time.

In an analytic procedure such as the fluorescence micelle assay it is useful if the preparation of the samples before the actual fluorescence micelle assay measurement is carried out as quickly as possible so that there is not a large time difference between the individual samples, for example, resulting in different contact times with the dye. Therefore, immediately after the addition of the dye solution, all samples are incubated as soon as possible. In any case, it may be useful if the dye-containing samples are protected from direct exposure to light prior to performing the fluorescence micelle assay.

In method step (a.4) a known fluorescence micelle assay of the sample obtained in step (a.2) or step (a.3) is performed. Since step (a.3) is an optional step it is not always performed. In case step (a.3) is omitted, the fluorescence micelle assay of the sample obtained in step (a.2) is performed, in case step (a.3) is employed, the fluorescence micelle assay of the sample obtained in step (a.3) is performed. In the fluorescence micelle assay light of a wavelength of 350 nm is used to excite the fluorescence dye which then emits a fluorescence light of the wavelength of 420 nm. A spectrofluorometer is used to carry out the fluorescence measurement. Also a known sample container may be used for the measurement such as a quartz cuvette. It is advantageous if the sample container or receptacle used in steps (a.1), (a.2), and (a.3) is the same in which also the fluorescence measurement in step (a.4) is conducted.

Thus, the method (a) is performed with the use of a fluorescence detector, a HPLC system or device is not used.

Using the fluorescence micelle assay the content of polysorbate present in the sample is determined in step (a.5). For quantification, the fluorescence signal can be evaluated with the help of a calibration curve, a blank value and a suitable software can be used to determine the quantity of the polysorbate.

Thus, the method (a) can be applied for polysorbate content determination in (poly)peptide(s) containing aqueous formulations without the need of a previous (poly)peptide(s) removal step.

In method (a) steps (a.1), (a.2), (a.3), (a.4), and (a.5) may be carried out one after the other, in the order mentioned, for example directly one after the other. It can be useful if there are no intermediate steps between the individual method steps beside the aspects already described.

In case several samples shall be evaluated it is advisable if the samples may be processed in parallel or simultaneously.

Further, method (a) allows to significantly reduce the analysis time per sample from 120 s of the FMA-HPLC method to about 15 s - 20 s for the fluorescense micelle assay, thus reducing the analysis time by a factor of at least 6.

It is possible that a part of the method steps or all method steps may be selected to be automated.

According to the present invention, a method according to the appended claims may be used for a quantification screening of polysorbate in aqueous formulations of a great number of samples, particularly at the same time. The method may be adapted so that it can be performed using a liquid microbatch format combined with several liquid-handling steps for high-throughput analysis purposes.

Compared to method (a) as described herein, in the method of the present invention a number of samples, especially a great number, of an aqueous formulation containing polysorbate and one or more (poly)peptides are provided in step (i).

The number of the samples which can be provided in step (i) depends from several factors, such as the type of sampling, the size and type of sampling device used, the desired sample throughput, the size and type of the fluorescence micelle assay device and the like. By way of example, the number of samples can be specified with at least 2, 5, 10, 20, 50, 96, 100, 200, 384, 500 or 1000. Also another number of samples may be provided in step (i).

In the method step (i) the samples of the aqueous formulation to be tested may be provided at the same time (simultaneously) or the samples to be tested may be provided one after the other. If the samples are provided simultaneously, the throughput may be significantly increased.

According to the invention the method is performed in a microplate format. The expression "microplate format" is to be understood in that the number of samples to be quantified is provided in microplates (also known as microtiterplates), each sample is provided in one well of the microplate. A microplate is a well-known laboratory device for the examination of biological or physical properties. The mostly rectangular microplates are usually made of plastic and contain many wells isolated from each other in rows and columns. A microplate typically has 6, 12, 24, 48, 96, 384 or 1536 sample wells; also microplates with 3456 or 9600 wells are known. Each well of a microplate may typically receive and contain a volume of several tens of nanolitres to several millilitres of the liquid sample.

Such microplates are particularly suitable for a high-throughput screening in pharmaceutical investigations. Therefore, the method of the present invention can be adapted to be used in a 'microplate formate' because the method may be performed using a microplate or several microplates.

In order to achieve a high-throughput method a part of the method steps or all method steps of the method of the invention may be selected to be automated.

According to an embodiment step (i) is automated. Therefore, according to an embodiment each sample to be tested is transferred into a well of a microplate by an automated device or system. For example, a number of samples may be processed in parallel and at the same time. There are no particular restrictions on the number of samples that can be processed simultaneously, only the type and size of the automated processing station used or the size of the microplate used can limit the number of samples to be examined.

Therefore, for example, a liquid handling device or station may be used which can process a great part or all samples simultaneously in parallel. In such a case a microplate having a defined number of wells is used in connection with a robotic workstation having equipment to automatically take and process analytic samples such as liquid flexible channel arms, liquid handling arms equipped with disposable or fixed tips for multi-pipetting, robotic grip arms and the like. Such robotic workstations usually also have integrated control systems such as a liquid-level detection function or similar functions in order to ensure the correct processing of the samples.

Such automation has great advantages, as the sample volume of all samples to be analysed is exactly the same, the method steps per se and in combination are carried out as quickly as possible and many more samples can be processed simultaneously and in parallel.

In step (ii) the solution containing the fluorescent dye N-phenyl-1-naphthylamine is added to each of the samples, respectively, to obtain fluorescent dye containing samples to be tested in a subsequent fluorescence micelle assay. According to an embodiment the solution comprising the fluorescent dye N-phenyl-1-naphthylamine is simultaneously added to the samples which are present in a microplate, i.e. each sample is located in a well of the microplate. Then, the fluorescent dye N-phenyl-1-naphthylamine is present in each sample at the same time. Therefore, a variety of samples may be processed at the same time. It is also possible to add the fluorescent dye N-phenyl-1-naphthylamine to each of the samples one after the other. Apart from that the explanations to step (a.2) apply equally here. Especially, according to an embodiment the solution containing the dye in step (ii) may have the same composition as used for the mobile phase in a combined FMA-HPLC method as described in the prior art (see [9] loc.cit.]

It is advantageous if step (ii) is automated because an equal treatment of all samples is ensured.

Prior to adding the solution comprising the fluorescent dye N-phenyl-1-naphthylamine to the samples, it may be useful to mix the solution. For example, the solution is automatically shaken on a shaker to avoid concentration inhomogeneity of the dye due to unspecific adsorption on the surface of the reservoir.

Subsequently, in method step (ii'), the samples obtained in step (ii) may be incubated. This represents an optional method step. The incubating may be performed under shaking. For example, the incubating in step (ii') may be performed with or without shaking, which is realized for the samples at the same time or one after the other.

According to an embodiment the incubating temperature is selected from the range of from 10 to 60 °C, particularly 30 to 40 °C, more particularly 35 °C. An incubation step, particularly under shaking, may have the advantage that a partitioning of the fluorescence dye into the inner core of the polysorbate micelles might be increased.

The incubation time may be readily selected by the skilled person. However, in the present invention the incubation time may be adjusted to be very short as already explained. Also shorter or longer incubation times are possible. The skilled person is readily able to select a suitable incubation temperature in the above range and to select an incubation time in accordance with his knowledge.

According to an embodiment also optional step (ii') may be automated. It is advantageous if step (ii') is automated because an equal incubation temperature and time and an equal shaking performance and time of all samples can be ensured.

The shaking during incubation may be performed by an incubator shaker, for example. Such incubator shakers are known temperature control instruments combining incubation and shaking functions.

After incubation and before performing the fluorescence measurements for quantification, it is not necessary to bring the samples to room temperature.

Subsequently, the fluorescence intensity of the samples obtained in method step (ii) (if no incubation step takes place) or obtained in method step (ii') (if an incubation step takes place) is measured spectrophotometrically in a fluorescence micelle assay in step (iii) with an excitation wavelength of 350 nm and an emission wavelength of 420 nm.

According to an embodiment also step (iii) may be automated. This is advantageous because the fluorescence micelle assay may be performed as quickly as possible with a variety of samples.

Using the fluorescence micelle assay the content of polysorbate present in each of the samples is determined in step (iv) by using a calibration curve. According to an embodiment also step (iv) may be automated.

The method of the invention is performed with the use of a fluorescence detector, a HPLC system or device is not used.

According to an embodiment the method steps (i) to (iv) may be conducted fully automated whereby several samples or all samples are processed in parallel. The inventive method then allows to perform a fully automated high-throughput fluorescence micelle assay, for example, conducted in a microplate format using a liquid-handling station. This approach significantly reduces the analysis time per sample from 120 s of the FMA-HPLC method to about 15 s - 20 s for the fluorescense micelle assay performed in the method of the invention, thus reducing the analysis time by a factor of at least 6.

In Figure 5 a schematic illustration of the method according to steps (i) to (iv) according to an embodiment of the present invention is shown and described in the following:
In step (i) a number of samples 210.1, 210.2, 210.3, and 210.4 of an aqueous formulation containing polysorbate and one or more polypeptides such as one or more polypeptides, or proteins are provided. The polysorbate has formed micelles 220a, 220b, and 220c, which are depicted as black spheres, so that the concentration of the polysorbate is equal or higher than the critical micelle concentration (c(PS) ≥ CMC). In the shown exemplary embodiment the samples 210.1, 210.2, 210.3, and 210.4 are present in the wells of a microplate, i.e. each sample is present in one well of the microplate, respectively. That is, the samples 210.1, 210.2, 210.3, and 210.4 in step (i) have been already transferred onto a microplate by an automated device or system, for example, a liquid handling device or station. A schematic illustration of an embodiment of a microplate 400 is shown in Figure 5A. Other embodiments of a microplate are also possible.
In step (ii) in this exemplary embodiment the solution comprising the fluorescent dye N-phenyl-1-naphthylamine is simultaneously added to each of the samples 210.1, 210.2, 210.3, and 210.4, respectively, by an automated procedure. For example, the solution is simultaneously pipetted with suitable feeding systems 310.1, 310.2, 310.3, 310.4 by an automated system or device 300.

Prior to adding the dye containing solution it may be automatically mixed (not shown), for example shaken, to avoid concentration inhomogeneity of the dye.

In the exemplary embodiment shown the optional method step (ii') is performed and the samples are incubated with or without shaking. If a shaking is selected this may be performed, for example, by use of an incubator shaker. Such an incubator shaker may simultaneously hold and process one or more microplates, each containing a variety of samples. In the shown exemplary embodiment the incubating temperature is selected to be 35°C for 60 s so that the partitioning of the fluorescence dye into the inner core of the polysorbate micelles 220a, 220b, and 220c might be improved. Other incubation times or no incubation are conceivable and possible. The incubation time may be readily selected by the skilled person. The incubation temperature has been selected from the range as disclosed herein.

After incubation under shaking or without shaking, the fluorescence micelle assay of step (iii) of the samples obtained in method step (ii') is conducted in a spectrophotometer with an excitation wavelength of 350 nm (represented by arrow 350.1) and an emission wavelength of 420 nm (represented by arrow 350.2). Using the fluorescence micelle assay the content of polysorbate present in the sample is determined in step (iv) for example using appropriate software.

It has been found particularly advantageous to provide and test the samples in microplates, as the microplates are also suitable for high-throughput screening in a spectrophotometer. Thus, the method of the present invention including the testing of the samples can be performed partly or fully automated, e.g. on a liquid-handling station, whereby the use of a microplate format is beneficially.

In the following the term "methods" shall refer to both the non-claimed method (a) according to steps (a.1) to (a.5) as described herein and the method according to the present invention according to steps (i) to (iv), unless otherwise stated.

The methods may be performed using disposable labware such as disposable microplates. This prevents carry-over from one sample to another and cleaning problems with the equipment do not occur.

The methods may be performed using disposable labware which has non-binding, low-binding or medium-binding surfaces. In order to minimise the binding of the fluorescent dye N-phenyl-1-naphthylamine, the use of non-binding, low-binding or medium binding surfaces may be useful. For example, several types of polystyrene microplates are known which have non-binding, low-binding or medium-binding surfaces for said fluorescent dye.

Such specific, non-binding disposable labware, can overcome the above-mentioned disadvantages of the fluorescence micelle assay performed on a HPLC system like dedication of analytical equipment which is adapted for the special use and also fluorescence signal fluctuations caused by adsorption and desorption events of the hydrophobic dye N-phenyl-1-naphthylamine in the HPLC system can be circumvented.

The polysorbate concentrations in the samples may be readily determined whereby the methods allow the analysis of nearly all types of aqueous formulations. If the concentration of polysorbate is to be determined in samples with a very low or very high concentration of polysorbate, it may be useful to adjust the sample volume for these samples, e.g. by an additional dilution step or by concentrating the sample. Adjusting the sample volume might also be necessary before analysis to fit into the linear range of the calibration curve. The linear range of the calibration curves may be influenced by various experimental parameters, e.g. the batch-by-batch composition of the polysorbate used. However, the skilled person can readily perform a calibration with suitable calibration samples for each polysorbate to be used, determine the linear range and take the results into account in the measurements, based on his knowledge and the technical teaching disclosed herein.

It is therefore appropriate if the results of the calibration samples, for given linearity, are used for subsequent evaluation of the polysorbate content of a series of samples to be measured.

As will be demonstrated in detail, the methods are also highly suitable for biopharmaceutical samples and show accurate and specific results for the determined polysorbate content. The high precision of the methods found, also if a variety of samples is screened, proves the non-existence of random errors, which might be traced back to the use of several liquid-handling steps, also in view of an automated liquid-handling station.

It has been found that the methods are very selective and show no or very low interferences due to hydrophobic components potentially present in the aqueous formulations evaluated. Even polypeptide(s) such as polypeptides or proteins with different hydrophobic properties do not impair the method in any way. This is unexpected.

In addition, the polysorbate quantification methods are applied to analyse polypeptide or protein containing samples. Actually, the presence of a polypeptide, particularly a protein, in the aqueous formulation has no negative influence on the methods of the invention. The fluorescence micelle assay performed according to the methods is usable in the presence of polypeptide(s), particularly polypeptide(s) or protein(s). The hydrophobic fluorescence dye N-phenyl-1-naphthylamine has not been found to interact with other hydrophobic components within the aqueous formulations such as peptides, polypeptides, or proteins. Further, it has been found that the known auto- or self-fluorescence of components within the aqueous formulations, for example of peptides, polypeptides, or proteins, did not result in an unwanted excitation of N-phenyl-1-naphthylamine (at a wavelength of 350 nm) and no errors in the quantification of polysorbate could be found due to such an interaction. Therefore, the well-established finding in prior art that the fluorescence micelle assay shall not be possible in the presence of a protein is not correct and not in accordance with our experimentations to be explained later.

Also a nonuniformity of the interaction of N-phenyl-1-naphthylamine with different polysorbates could not be observed so that a misestimation of the polysorbate content did not occur.

The polysorbate quantification methods can therefore and unexpectedly applied to analyse polypeptide(s) or protein(s) containing samples; in other words, the fluorescence micelle assay performed in the methods allows to quantify polysorbate in the presence of polypeptide(s) or protein(s). As a result, a preparative polypeptide- or protein-removal step is needless at all.

The methods can be performed with one or several samples, also a high number of samples may be evaluated. The methods may be conducted by hand or by an automated system. The method steps may be perfomed one after the other and also at the same time in parallel in order to save time.

Furthermore, the method of the invention, based on a fluorescence micelle assay using the dye N-phenyl-1-naphthylamine, is implemented in microplate format and is therefore suitable for high-throughput concentration screening.

In the method of this invention not using a HPLC system, the analysis time per sample can be reduced from 2 min per sample when performing the fluorescence micelle assay on a HPLC system to about 15 - 20 s per sample in the inventive method, thus enabling an increased sample throughput. Alternative polysorbate quantification methods, such as UPLC-QDa (Ultra Performance Liquid Chromatography with Quadrupole Dalton detection) has an analysis time of 10 min per sample [13], mixed-mode HPLC-CAD (High-Performance Liquid Chromatography with Charged Aerosol Detection) has an analysis time of 8 min per sample, and reversed-phase (RP) UHPLC-CAD (Ultra High Performance Liquid Chromatography with Charged Aerosol Detection) has an analysis time of 36 min [3] so that the method of the present invention is clearly superior to state of the art methods in terms of the analysis time required.

Thus, by the transfer of the assay from the FMA-HPLC system to the microplate format, for example, executed on a liquid-handling station, particularly according to the method of the invention, the analysis time per sample can be reduced by a factor of at least six. This reduction of analysis time is highly advantageous for large sample sets and makes the method according to the present invention superior compared to other polysorbate analytical methods with longer analysis times. Particularly such a reduction of analysis time is very valuable in industrial scale, for example during the development of biotherapeutic products.

There is therefore provided a simple fluorescence-based method for the rapid determination of the polysorbate content in aqueous formulations which may be also very suitable in industrial scale.

In fact, the described method of the invention enables the screening of a much larger number of samples, thus, providing more robust and reliable data on polysorbate content and polysorbate degradation that may be present. The large throughput also enables the analysis of sample replicates and therefore allows a statistical interpretation of the collected data.

With regard to the sample volume to be checked there are no special restrictions, the sample volume can be very low and is comparable with the combined fluorescence micelle assay (FMA)-HPLC setup of the prior art. For example, a sample having an amount of 10 µL may be examined in the method of the invention.

Since the accuracy and reliability of the quantification of polysorbate by means of the fluorescence micelle assay has been questioned or even doubted in some publications, it is in any case necessary to examine the fluorescence micelle assay as an analytical method in this respect in detail.

In order to verify whether the fluorescence micelle assay performed on a liquid-handling station in a microplate format is a reliable test method, the assay was investigated in terms of linearity, specificity, accuracy, robustness, and precision according to literature [14, 15]. The detailed experiments are described in the experimental section. The method of the invention was assessed in terms of these parameters for the determination of surfactant concentrations in samples in the presence or absence of polypeptides, particularly polypeptides or proteins. The essentials of these studies are summarized in the following:

### Linearity

The ICH Q2(R1) guideline was followed [14], according to which the linearity of an analytical method indicates its ability, within a certain range, to produce results directly proportional to the concentration of the analyte in the sample. For the testing of the linearity between the fluorescence signal and the polysorbate concentration, the fluorescence was measured for samples containing different set polysorbate concentrations (see the experimental section). The linearity of the fluorescence signal for the fluorescence micelle assay (FMA) performed in microplates was demonstrated for concentrations up to 0.6 mg/mL PS20 HP (polysorbate 20 high purity), up to 0.8 mg/mL polysorbate 20 China grade (PS20 China grade), and up to 0.3 mg/mL PS80 HP (polysorbate 80 high purity).

Typically, biopharmaceutical formulations contain polysorbate concentrations in the above-mentioned ranges and can therefore be measured directly using the method of the present invention. For samples containing higher or lower concentrations of polysorbate, the sample volume can be adjusted as required, for example a dilution step or concentration step may be conducted prior to performing the method of the present invention.

### Specificity

Specificity refers to the ability to unambiguously determine the analyte in the presence of expected components such as impurities, degradation products, matrix, etc. (see literature [17]). In the fluorescence micelle assay, this means that the fluorescence signal is only due to the polysorbate present, but no fluorescence signal comes from the polypeptide or protein, the buffer or the added excipients. In terms of specificity, different monoclonal antibodies (mAbs) have been examined, the most of them did not show a fluorescence signal and no problems were encountered with regard to specificity of the fluorescence signal from polysorbate in the presence of these polypeptides or proteins.

Another protein in form of a monoclonal antibody (mAb) showed an intrinsic fluorescence signal observed at the wavelengths used for the fluorescence micelle assay. In the absence of polysorbate this florescence signal of the protein per se was referred to as 'apparent' polysorbate concentration. This 'apparent' polysorbate concentration of the protein (in the absence of polysorbate) showed a fraction of the intensity of the fluorescence signal of the polysorbate which was determined in a separate experiment. In the experiment as shown in the experimental section the protein showed a fluorescence signal which corresponds to 15 % of the total fluorescence signal of the polysorbate concentration of the formulated sample. Surprisingly, despite the presence of both components - protein and polysorbate - whereby the protein alone showed a fluorescence signal in the fluorescence micelle assay, only the polysorbate was selectively detected with the fluorescence micelle assay. There was no signal intensification derived from the protein.

That means that even if a peptide, polypeptide, particularly a protein, in the sample shows an intrinsic fluorescence signal in the absence of polysorbate, the fluorescence micelle assay is capable to selectively detect the signal arising from the polysorbate when both components are present.

The high specificity of the assay was also shown in connection with a widely used standard protein, namely alpha-lactalbumin. This standard protein has a molecular mass of about 10 % of a mAb and a significantly lower complexity in its structure. In contrast to other mAbs which have been examined, alpha-lactalbumin has an acidic pl (isoelectric point), and therefore a negative overall surface charge under the experimental conditions used, while the other mAbs used had a positive net charge under the experimental conditions applied. In control samples composed of pure buffers and pure formulation buffers without spiked polysorbate no interfering signal derived from the buffer components and the added excipients has been observed. These results support our finding, that the fluorescence micelle assay is very specific for the analysis of polysorbate, independent of the physicochemical properties of the peptides, polypeptides, or proteins, buffer or excipient substances present in the solution.

The high specificity of the assay was also shown in connection with a widely used enzyme, namely lysozyme. Lysozyme is a protein and enzyme which consists of 129 amino acids and molecular weight thereof is approximately 14 kDa to 15 kDa. Lysozyme has an alkaline pl (isoelectric point) and therefore a positive surface charge under the experimental conditions used, as the mAbs used. Also these results support our finding, that the fluorescence micelle assay is very specific for the analysis of polysorbate, independent of the physicochemical properties of the peptides, polypeptides, or proteins, buffer or excipient substances present in the solution.

### Accuracy

The accuracy of an analytical procedure is expressed in the degree of agreement between the value, which is either a conventional true value or an accepted reference value, and the value found [14]. As demonstrated in the experimental section, in terms of accuracy, the calculated spike recovery rates of polysorbate were in a range of 80 % and 117 % for PS20 HP (polysorbate 20 high purity) spiked in buffer without protein. For the samples containing protein, buffer substances and either PS20 HP (polysorbate 20 high purity) or PS80 HP (polysorbate 80 high purity), the accuracy was in a range of 73 % and 117 % for the investigated formulations with PS20 HP (polysorbate 20 high purity) and 79 % - 118 % for the formulations containing PS80 HP (polysorbate 80 high purity).

Based on our experience, this range is comparable with other analytical methods and can be regarded as acceptable for the analysis of samples, particularly biopharmaceutical samples.

At the low concentration of 0.1 mg/mL PS20 HP (polysorbate 20 high purity), it can be that the fluorescence micelle assay shows the tendency to determine slightly lower values than the target concentration. This observation could be caused by a non-linear correlation between the polysorbate concentration and the fluorescence signal at polysorbate concentrations in a concentration range near the critical micelle concentration (CMC).

### Precision

The precision of an analytical procedure expresses the closeness of agreement (degree of scatter) between a series of measurements obtained from multiple sampling of the same homogeneous sample under the prescribed conditions [17]. For the fluorescence micelle assay performed on a liquid-handling station, the precision was tested for five different mAbs in the experiments under the respective formulation conditions containing the target polysorbate in high purity (PS HP) quality. Every sample was analyzed multiple times, each as a 4x technical replicate. For the investigation of the precision, the average %CV (coefficient of variation, also known as relative standard deviation of all four technical replicates, that have been analyzed multiple times, is in a range of 2.2 % - 3.6 %. The maximum %CV obtained during the analysis of the precision for the five different mAbs were between 3.5 % and 6.8 %. The highest average %CV and the maximum %CV was observed for the samples containing a mAb and PS80 HP (polysorbate 80 high purity). This finding could indicate a lower precision of the fluorescence micelle assay for PS80 HP.

Overall, the results of the parameters to assess the capabilities of the fluorescence micelle assay indicate that the high-throughput fluorescence micelle assay is specific for the analyte polysorbate and shows a high precision and is, in combination with the acceptable accuracy, well suited for the determination of the polysorbate 20 and polysorbate 80 concentration present in samples containing (poly)peptide(s) or placebos.

### LOD and LOQ

For the fluorescence micelle assay, particularly conducted in microbatch format, the Limit of Detection (LOD) and the Limit of Quantitation (LOQ) were determined according to [17]. These terms are used to describe the smallest concentration of a measurand - here: the polysorbate concentration - that can be reliably measured by an analytical procedure. Typically, a test is simply not capable of accurately measuring analyte concentrations down to zero. Sufficient analyte concentration must be present to produce an analytical signal that can reliably be distinguished from "analytical noise", the signal produced in the absence of analyte. Various analytical specifications can be applied to ensure that the LOD is meaningful and clearly distinguishable from a negative or blank sample. In this regard it is also referred to the experimental section.

The following LODs and LOQs have been found:
The limit of detection (LOD) was determined to be 0.023 mg/mL of PS20 HP (polysorbate 20 high purity) and the limit of quantitation (LOQ) was determined to be 0.069 mg/mL of PS20 HP (polysorbate 20 high purity).

In contrast to PS20 HP (polysorbate 20 high purity), for the PS20 China grade (polysorbate 20 China grade) the LOD was determined to 0.009 mg/mL and the LOQ was 0.028 mg/mL, respectively. For PS80 HP (polysorbate 80 high purity) the determined values were 0.001 mg/mL of PS80 HP (polysorbate 80 high purity) for the LOD and 0.003 mg/mL PS80 HP (polysorbate 80 high purity) for the LOQ, respectively.

Comparing the determined LOD and LOQ of the method of the present invention, namely in view of an automated microplate based fluorescence micelle assay, and the method according to prior art using HPLC-FMA, the LOD/LOQ for PS20 HP (polysorbate 20 high purity) was in the same concentration range for both assay setups. For PS80 HP (polysorbate 80 high purity), the LOD and LOQ were significantly lower for the automated microplate based fluorescence micelle assay (FMA). However, Fekete at al. [16] determined for the PS80 HP (polysorbate 80 high purity) quantification via RP-HPLC in combination with a CAD detector (CAD: charged aerosol detection) a LOD and LOQ being 0.005 mg/mL and 0.01 mg/mL for polysorbate 80 (PS80), respectively. The comparison of these values with the LOD/LOQ obtained for the method of the present invention shows that the LOD/LOQ are in a similar range and the fluorescence micelle assay also has the potential to determine the polysorbate 80 (PS80) concentration in a range, that is below polysorbate 80 concentrations typically used in biopharmaceutical formulations.

Combining the results of the linearity and the LOD/LOQ determination, the applicability of the method of the present invention, namely for the microplate based fluorescence micelle assay, can be set to a range of 0.1 mg/mL - 0.6 mg/mL for samples containing PS20 HP (polysorbate 20 high purity), 0.1 mg/mL - 0.8 mg/mL for samples containing PS20 China grade (polysorbate 20 China grade), and 0.05 mg/mL - 0.3 mg/mL for samples containing PS80 HP (polysorbate 80 high purity).

According to the invention, a selective and easy-to-perform method is therefore obtained without time-consuming sample preparation, e.g. for the quantification of polysorbate 20 and polysorbate 80, in pharmaceutically relevant amounts in the presence of peptide(s), polypeptide(s), or protein(s) and without interference with components or excipients. In particular, a high-throughput modification according to method of the inventionis provided.

The invention is also directed to the use of the method according to the invention for the quantification of polysorbate in an aqueous formulation, for example as a stability test of the aqueous formulation, during the development, manufacture, shelf life, or storage time thereof, whereby the aqueous formulation contains polysorbate and one or more polypeptides as disclosed herein.

The advantages of the present invention are manifold:
The method according to the present invention has been developed in order to overcome the drawbacks of prior art, especially the fluorescence micelle assay performed on a HPLC-system has numerous disadvantages. This was successful.

The polysorbate quantification method of the present invention is applied for the first time to analyse polypeptide(s) containing samples. The method is also highly suitable for biopharmaceutical samples and shows accurate and specific results for the determined polysorbate content, even in the event one or more polypeptides are present.

The polysorbate concentrations in aqueous samples may be readily determined whereby the inventive methods allow the analysis of nearly all types of aqueous samples, also biopharmaceutic samples. If limitations in view of samples having concentrations being very low or very high occur, for these samples an adjustment of the sample volume is possible such as an additional dilution step or concentration step. The high precision of the method found, also if a variety of samples is screened, proves the non-existence of random errors, which might be traced back to the use of several liquid-handling steps, even when an automated liquid-handling station is used.

According to an embodiment the method is performed using disposable labware. This prevents carry-over from one sample to another and cleaning problems with the equipment do not occur.

In another embodiment the method is performed using disposable non-binding, low-binding or medium-binding labware for which the binding of the fluorescent dye N-phenyl-1-naphthylamine is minimised. With such specific, non-binding disposable labware, the disadvantages of fluorescence micelle assays performed with HPLC system, especially special adapted analytical equipment and fluorescence signal fluctuations caused by adsorption and desorption events of the hydrophobic dye N-phenyl-1-naphthylamine in the HPLC system can be avoided.

The method of the invention can be performed with several samples, also a high number of samples may be evaluated. The method is very flexible and may be conducted by hand or by an automated system. According to an embodiment the inventive method allows to perform a fully automated high-throughput fluorescence micelle assay, for example, conducted in a microplate format using a liquid-handling station.

The method steps may be performed one after the other and also at the same time in parallel in order to save time. In the inventive method the analysis time can be clearly reduced to about 15 to about 20 s per sample compared to 120 s when performing the fluorescence micelle assay on a HPLC system, thus reducing the analysis time by a factor of at least 6. This reduction of analysis time is highly advantageous, particularly for large sample sets, and makes the method according to the present invention superior compared to other polysorbate analytical methods with longer analysis times. Particularly the use of the method steps (i) to (iv) according to the invention performed in industrial scale, for example during the development of aqueous formulations such as biotherapeutic formulations or products, is extremely beneficial.

A part or all method steps may be automated. Particularly by transferring the method into a microbatch format and combining it with the benefits of a number of easy-to-perform liquid-handling steps, the entire procedure can be carried out in a simple, fully automated way, even with a large number of samples.

The sample volume to be used is generally very low and comparable with the combined FMA-HPLC setup of the prior art, for example a sample having an amount of 10 µL may be examined.

The preparation of the samples is not complex, apart from the addition of the dye with the sample, no further pre-treatment is required. The incubation step is only optional. The interaction between dye and polysorbate can be used in a simple way to perform quantification, especially in a high throughput method using multi-well plates.

In case a set of samples is to be tested at the same time a liquid-handling station may be used which allows performing parallelized and automated high-throughput analysis in a small scale [17].

Particularly the method of the invention enables the screening of a much larger number of samples, thus, providing more robust and reliable data on polysorbate content and polysorbate degradation that may be present. The large throughput also enables the analysis of sample replicates and therefore allows a statistical interpretation of the collected data.

The inventive method provided is sensitive, selective, and fast; does not require expensive or delicate instrumentation; and is also suitable for high-throughput, particularly due to the microplate format.

The determined LOD/LOQ allow the analysis of a broad set of samples, as polysorbate concentrations in aqueous samples are typically many times above these values. The linear range of the fluorescence micelle assay also allows the analysis of nearly all biopharmaceutical samples. Limitations could occur for formulations with a PS80 HP content above 0.3 mg/mL. For these samples the adjustment of the sample volume might be required such as an additional dilution step.

The method provided is highly appropriate for the quantification of polysorbates in aqueous formulations which has been demonstrated by determining the linearity, specificity, accuracy, robustness, as well as precision of several embodiments according to the invention.

The results also demonstrate the suitability in a high degree of the developed method to be used as high-throughput method carried out in a microplate format to screen large sample sets for the quantification of polysorbate, also in the presence of polypeptides and common excipients used for aqueous formulations. The results of the experiments support that the method of the invention is very specific for the analysis of polysorbate, independent of the physicochemical properties of the polypeptides, buffer or excipient substances present in the solution.

### List of abbreviations

| | |
|---|---|
| API | active pharmaceutical ingredient |
| CAD | charged aerosol detection |
| CMC | critical micelle concentration |
| CV | coefficient of variation |
| DF | diafiltration |
| FCA | flexible channel arm |
| FMA | fluorescence micelle assay |
| HIC | hydrophobic interaction chromatography |
| HPLC | high-performance liquid chromatography |
| IgG | immunoglobulin G |
| kDa | Kilodalton |
| LOD | limit of detection |
| LOQ | limit of quantification |
| NPN | *N*-phenyl-1-naphthylamine |
| PS | polysorbate |
| PS20 | polysorbate 20 |
| PS80 | polysorbate 80 |
| PS20 HP | polysorbate 20 high purity (commercial product) |
| PS80 HP | polysorbate 80 high purity (commercial product) |
| mAb | monoclonal antibody |
| PBS | phosphate buffered saline |
| PES | polyethersulfone |
| RP | reversed-phase |
| SPE | solid phase extraction |
| UF | ultrafiltration |

In the following the experimentations performed are illustrated. The features and advantages of the present invention will become apparent from the following detailed experiments, which illustrate some principles of the invention by way of example without limiting its scope.

### EXPERIMENTS

### 1. Materials and methods

### 1.1 Materials and reagents

All experiments were conducted with PS20 HP (polysorbate 20 high purity) and PS80 HP (polysorbate 80 high purity) from Croda Europe Ltd (Snaith, United Kingdom) and PS20 China grade (polysorbate 20 China grade) (Nanjing Well Pharmaceutical Co., Ltd, Nanjing, China).

Polysorbate stock solutions with a concentration of 100 mg/mL were prepared by adding the surfactant to Milli-Q^{®} water with a subsequent homogenization by using a magnetic stirrer. Milli-Q water^{®} is water provided by a water treatment system that produces both ultra-pure water (type 1) and pure water (type 2) of the highest quality directly from tap water. In the present experiments the water of type 1 has been used. If required the polysorbate stock solutions were further diluted with Milli-Q^{®} water, e.g., for the preparation of calibration samples.

The buffers used have a composition which is usally in protein reconditioning and further processing methods such as UF/DF (Ultrafiltration/Diafiltration) steps.

The proteins in form of several monoclonal antibodies (mAbs) were kindly provided by Boehringer Ingelheim Pharma GmbH & Co. KG. Alpha-lactalbumin from bovine milk type III ≥85 % and lysozyme were purchased from Merck KGaA (Darmstadt, Germany). The molecular properties of the proteins investigated in these experiments are listed in Table 1.

All experiments were carried out in black 96-well FLUOTRAC^{™} 200 polystyrene microplates (Greiner Bio-One GmbH, Frickenhausen, Germany).

The hydrophobic dye *N*-Phenyl-1-Naphthylamin (NPN), reagent grade (Sigma-Aldrich Corp., St. Louis, USA) was prepared with a concentration of 5 µM in a buffer containing 5 % (v/v) acetonitrile HPLC Plus grade ≥99.9 % (Sigma-Aldrich), 150 mM sodium chloride for analysis EMSURE^{®} (Merck KGaA, Darmstadt, Germany), 50 mM Trizma^{®} base ≥99.9 % (Sigma-Aldrich) and 0.0015 % Brij-35 MQ200 quality (Sigma-Aldrich) at pH 8. The buffer was filtered after preparation with a 0.22 µm Steritop^{®} PES filter (Merck) and stored protected from light at ambient temperature for a maximum of 7 days.

### 1.2 Protocol for FMA on a liquid handling station

The FMA was performed on a Tecan Fluent^{®} 1080 liquid handling station (Tecan Group, Männedorf, Switzerland). The platform was equipped with an 8-tip Flexible Channel Arm (FCA), a 96-channel liquid handling arm, a Robotic Gripper Arm, a BioShake 3000 elm orbital shaker (Quantifoil Instruments GmbH, Jena, Germany), an Incubator Shaker DWP (INHECO GmbH, Martinsried, Germany), and an Infinite^{®} M Nano+ dual-mode plate reader. All pipetting steps were performed with disposable tips (Tecan Group).

10 µL of each sample was transferred into the 96-well microplate by the FCA using a liquid-level detection function. To ensure an equal incubation time of all samples, 240 µL of the FMA buffer was added with the 96-channel liquid handling arm. Prior to adding the FMA buffer to the samples, the buffer reservoir was automatically shaken at 300 rpm for 60 s at room temperature on the orbital shaker to avoid concentration inhomogeneity of NPN due to unspecific adsorption on the surface of the reservoir. Subsequently, the plate was incubated for 60 s at 1000 rpm and 35°C for a possible better partitioning of the fluorescence dye into the inner core of the PS micelles. After incubation, the fluorescence intensity was measured spectrophotometrically with an excitation wavelength of 350 nm and an emission wavelength of 420 nm. All samples were analyzed as four technical replicates with our current experimental setting. A technical replicate means the repeated measurement of the same sample to assess the random noise associated with the protocol or equipment.

### 2. Results

### 2.1. General method development

By transferring the FMA from the HPLC system to the microplate format, the analysis time per sample could be reduced from 2 min to about 15 s to about 20 s, thus enabling an increased sample throughput. Polysorbate quantification methods require significantly increased analysis time per sample (for example: UPLC-QDa: 10 min per sample [13], mixed-mode HPLC-CAD: 8 min per sample, and reversed-phase (RP) UHPLC-CAD: 36 min per sample [3]).

### 2.2. Analysis of polysorbate content

The analytical method of the present invention was evaluated for the determination of PS20 HP, PS20 China grade, and PS80 HP content in aqueous solutions as well as in samples with biopharmaceutical protein solutions and formulations. The parameters evaluated were linearity, specificity, accuracy, and precision. Furthermore, the LOD and LOQ were determined.

### 2.2.1 Linearity

The linearity of the method of the present invention was evaluated according to the ICH Q2(R1) guideline [14], according to which the linearity of an analytical procedure is its ability (within a given range) to obtain results that are directly proportional to the concentration of analyte in the sample.

For the testing of the linearity between the fluorescence signal and the polysorbate concentration, the fluorescence was measured for samples containing different set polysorbate concentrations in a range of 0.05 mg/mL to 0.8 mg/mL polysorbate. As criteria for the linearity, a R² of the linear fit ≥0.99 was defined. The excitation wavelength was set to 350 nm and emitted light was measured at 420 nm. In Figure 6 the results for the linearity of the FMA are shown, i.e. the linearity of the fluorescence signal with increasing PS20 HP, PS20 China grade and PS80 HP concentrations is illustrated. With the above-mentioned constraint for R², the fluorescence signal was linear up to a content of 0.6 mg/mL PS20 HP and 0.8 mg/mL PS20 China grade. For PS80 HP, linearity was observed for concentrations up to 0.3 mg/mL. The linear range of the calibration curves may be influenced by various experimental parameters, e.g. the batch-by-batch composition of the polysorbate used. However, the skilled person can readily perform a calibration with suitable calibration samples for each polysorbate to be used, determine the linear range and take the results into account in the measurements, based on his knowledge and the technical teaching disclosed herein.

It is therefore appropriate if the results of the calibration samples, for given linearity, are used for subsequent evaluation of the polysorbate content of a series of samples to be measured.

### 2.2.2 Specificity

Specificity refers to the ability to assess unequivocally the analyte in the presence of components which may be expected to be present. Typically, these might include impurities, degradants, matrix etc. [14]. In the case of the fluorescence micelle assay, this means that there is no fluorescence signal resulting from the protein, the buffer substances, or the added excipients, except from the polysorbate present.

In the present experiments, five different mAbs were used, with different physicochemical characteristics, and the standard protein alpha-lactalbumin was used for investigating the specificity of the FMA. The detailed characteristics of these proteins are listed in Table 1. mAb1, mAb2, and mAb3 are IgGs of the subclass IgG1, while mAb4 and mAb5 belong to the IgG4 subclass. Only for mAb2 an intrinsic fluorescence signal at the wavelengths used in the FMA was detected, all other mAbs did not show an intrinsic fluorescence at the respective wavelength. In terms of protein hydrophobicity, there was a factor of 4.61 between the investigated proteins, while mAb4 was the least hydrophobic one and mAb2 the molecule with the most prominent hydrophobic character.

**Table 1: Overview of physicochemical properties of mAbs that were analyzed with high-throughput fluorescence micelle assay (experimental data not shown).**

| **Molecule** | **mAb1** | **mAb2** | **mAb3** | **mAb4** | **mAb5** | **Alpha-lactalbumin** | **Lysozyme** |
|---|---|---|---|---|---|---|---|
| **Format** | IgG1 | IgG1 | IgG1 | IgG4 | IgG4 | n/a | n/a |
| **Molecular weight** | 146 kDa | 145 kDa | 144 kDa | 145 kDa | 146 kDa | 14 kDa | 14 kDa |
| **Isoelectric point** | > 8 | 8.6 - 9.3 | 8.3 | 7.7 | 7.9 | 4.5 | 11.3 |
| **Self-fluorescence*** | No | Yes | No | No | No | No | No |
| **Protein concentration** | 150 mg/mL | 65 mg/mL | 100 mg/mL | 20 mg/mL | 20 mg/mL | 0.5 mg/mL | 10 mg/mL |
| **Protein hydrophobicity**** | 2.95 | 4.61 | 1.69 | 3.50 | 2.24 | 1 | n/a |
| **Polysorbate content** | 0.4 mg/mL PS20 | 0.2 mg/mL PS20 | 0.5 mg/mL PS80 | 0.4 mg/mL PS20 | 0.4 mg/mL PS20 | 0.2 mg/mL PS20 | ≤0.3 mg/mL PS80 |

| | | | | | | | |
|---|---|---|---|---|---|---|---|
| * Threshold value: >10 % of overall fluorescence signal derived from protein alone in buffer without polysorbate at formulation protein concentration determined as 'apparent' polysorbate concentration. ** Determined by HIC retention time, values normalized to retention time of alpha-lactalbumin. n/a not applicable | | | | | | | |

### - mAb1 (IgG1)

In the present experiments, the method according to the invention was carried out with a purified mAb1. The mAb1 was used in an acetate buffer system at pH 5.5 and in a formulation buffer system with acetate, sucrose and L-arginine at typical concentrations used for biopharmaceutical formulations at pH 5.5. The target mAb1 concentration in the formulation was 150 mg/mL. As polysorbate PS20 HP (polysorbate 20 high purity) was used.

Figure 7A shows the results of the specificity evaluation of the FMA. The samples examined were as follows:
- 176 mg/mL mAb1 in acetate buffer without polysorbate,
- 150 mg/mL mAb1 in acetate buffer without polysorbate
- pure acetate buffer without polysorbate
- pure formulation buffer without polysorbate
- formulation buffer + 0.4 mg/mL PS20 HP (polysorbate 20 high purity) and
- 150 mg/mL mAb1 in formulation buffer + 0.4 mg/mL PS20 HP (polysorbate 20 high purity).

The results demonstrated that there was no significant background fluorescence signal, and thus, no influence on the determined polysorbate concentration resulting from the protein, the acetate buffer (which is an UF/DF buffer: Ultrafiltration buffer/Diafiltration buffer), and the components of the formulation buffer without polysorbate. An acetate buffer is a buffer used, for example, in a UF/DF step, which is a well-known procedure in protein processing.

In the sample containing formulation buffer + 0.4 mg/mL PS20 HP (polysorbate 20 high purity), the PS20 HP concentration was determined to be 0.403±0.005 mg/mL PS20 HP.

In the sample containing 150 mg/mL mAb1 in formulation buffer + 0.4 mg/mL PS20 HP (polysorbate 20 high purity), the PS20 HP concentration was determined to be 0.394±0.018 mg/mL PS20 HP. The target PS20 HP concentration was 0.4 mg/mL PS20 HP.

### - mAb2 (IgG1)

In the present experiments, the method according to the invention was carried out with a purified mAb2. The purified mAb2 was presented in a histidine buffer system at pH 6.0 and in a formulation buffer system including histidine, sucrose, and mannitol at typical concentrations used for biopharmaceutical formulations at pH 6.0. The target mAb2 concentration in the formulation was 65 mg/mL. As polysorbate the PS20 HP (polysorbate 20 high purity) was used.

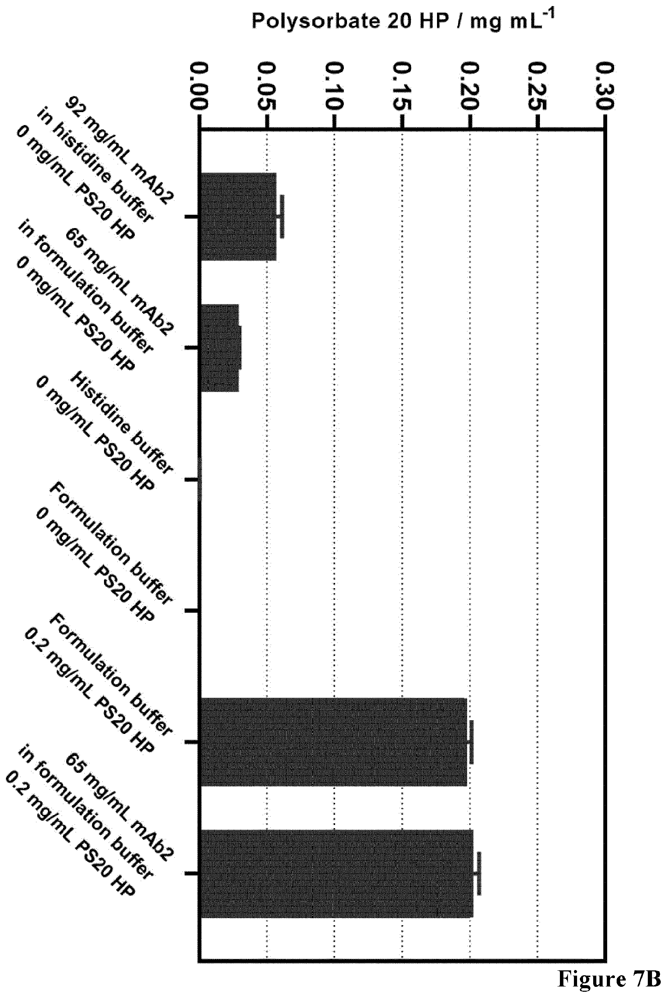

Figure 7B shows the results of the specificity evaluation of the FMA performed with the following samples:
- 92 mg/mL mAb2 in histidine buffer without polysorbate
- 65 mg/mL mAb2 in formulation buffer without polysorbate,
- pure histidine buffer without polysorbate
- pure formulation buffer without polysorbate
- formulation buffer + 0.2 mg/mL PS20 HP (polysorbate 20 high purity) and
- 65 mg/mL mAb2 in the formulation buffer + 0.2 mg/mL PS20 HP (polysorbate 20 high purity).

The results demonstrated that there was no significant background fluorescence signal, and thus, no influence on the determined polysorbate concentration, resulting from the histidine buffer (which is an UF/DF buffer: Ultrafilfration buffer /Diafiltration buffer) and the components of the formulation buffer without polysorbate. In contrast to mAb1, mAb2 showed an intrinsic fluorescence signal in the FMA with 0.057 mg/mL 'apparent' PS20 HP at a mAb2 concentration of 92 mg/mL (wherein no polysorbate was present) and 0.03 mg/mL 'apparent' PS20 HP at a mAb2 concentration of 65 mg/mL (wherein no polysorbate was present). This 'apparent' PS20 HP concentration under the later formulation condition of 65 mg/mL mAb2 in a buffer without added PS20 HP, was therefore solely derived from the mAb2. This constitutes about 15 % of the signal of the later mAb2 formulation in a buffer with 0.2 mg/mL PS20 HP.

In the sample containing formulation buffer + 0.2 mg/mL PS20 HP (polysorbate 20 high purity), the PS20 HP concentration was determined to be 0.198±0.003 mg/mL PS20 HP.

In the sample containing 65 mg/mL mAb2 in the final formulation buffer + 0.2 mg/mL PS20 HP (polysorbate 20 high purity), the PS20 HP concentration was determined to be 0.202±0.005 mg/mL PS20 HP. The target PS20 HP concentration was 0.2 mg/mL.

### - mAb3 (IgG1)

The mAb3 molecule is also an IgG1 format and was formulated in an acetate buffer system with the excipient trehalose with a surfactant concentration of 0.5 mg/mL PS80 HP at a high protein concentration of 100 mg/mL at pH 5.5. As the PS80 HP concentration was above the upper limit of the linear range (see 2.2.1 linearity: 0.3 mg/mL PS80 HP), samples were therefore diluted 1:2 with Milli-Q water before the analysis. As polysorbate the PS80 HP (polysorbate 80 high purity) was used.

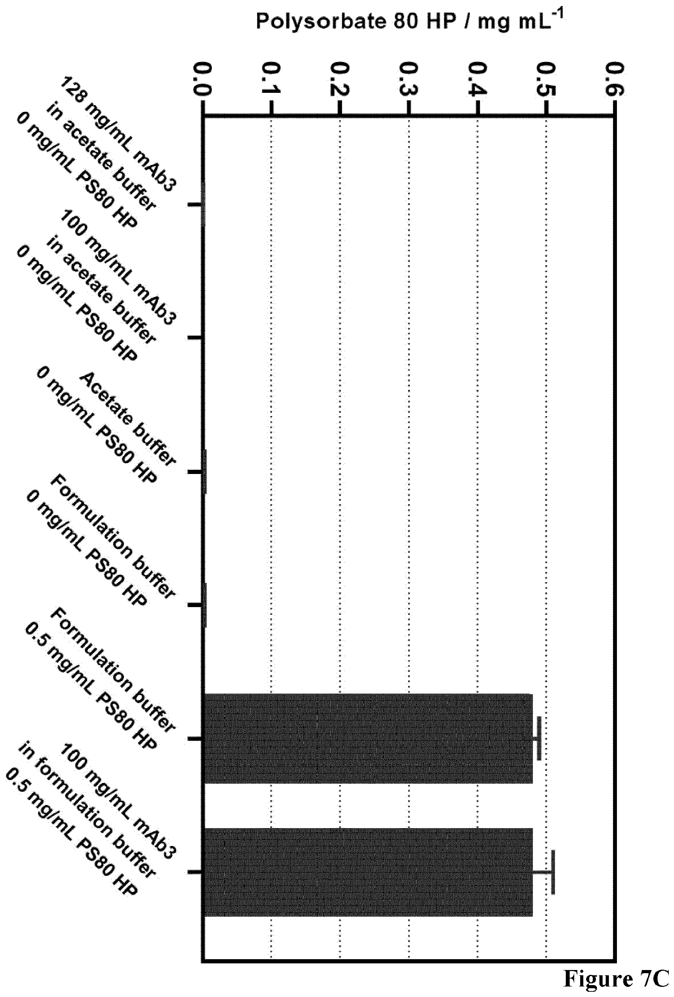

Figure 7C shows the results of the specificity evaluation of the FMA performed with the following samples:
- 128 mg/mL mAb3 in acetate buffer without polysorbate
- 100 mg/mL mAb3 in acetate buffer without polysorbate,
- pure acetate buffer without polysorbate
- pure formulation buffer without polysorbate
- formulation buffer + 0.5 mg/mL PS80 HP (polysorbate 80 high purity) and
- 100 mg/mL mAb3 in the formulation buffer + 0.5 mg/mL PS80 HP (polysorbate 80 high purity).

There was no significant fluorescence signal obtained from the FMA resulting from the samples without the presence of polysorbate, namely the pure acetate buffer (Ultrafiltration/Diafiltration buffer), the pure formulation buffer or mAb3 in acetate buffer at a protein concentration of 128 mg/mL or 100 mg/mL, respectively. That means the components present in the samples without polysorbate did not show a signal derived from the components present. The analysis of formulation buffer containing 0.5 mg/mL PS80 HP resulted in a determined PS80 HP concentration of 0.48±0.01 mg/mL PS80 HP. The final formulation, composed of 100 mg/mL mAb3 in formulation buffer, resulted in a determined PS80 HP content of 0.48±0.03 mg/mL PS80 HP in the FMA. The target PS80 HP concentration was 0.5 mg/mL.

### - mAb4 (IgG4)

The mAb4 molecule is an IgG4 format. As polysorbate the PS20 HP (polysorbate 20 high purity) was used.

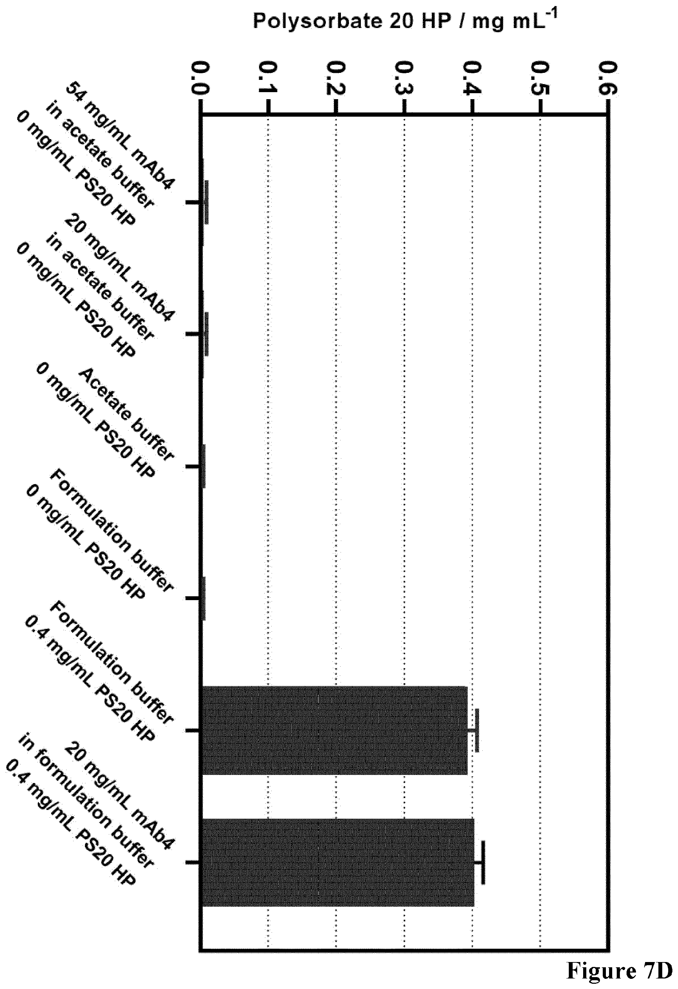

Figure 7D shows the results of the specificity evaluation of the FMA performed with the following samples:
- 54 mg/mL mAb4 in acetate buffer without polysorbate
- 20 mg/mL mAb4 in acetate buffer without polysorbate,
- pure acetate buffer without polysorbate
- pure formulation buffer without polysorbate
- formulation buffer + 0.4 mg/mL PS20 HP (polysorbate 20 high purity) and
- 20 mg/mL mAb4 in the formulation buffer + 0.4 mg/mL PS20 HP (polysorbate 20 high purity).

The specificity testing of the FMA for this molecule showed in the polysorbate-free samples no interfering signal derived from the pure acetate Ultrafiltration/Diafiltration (UF/DF) buffer and mAb4 at concentrations of 54 mg/mL and 20 mg/mL, respectively, in acetate buffer. An acetate buffer is a buffer used, for example, in a UF/DF step, which is a well-known procedure in protein processing. As for the other formulations, there was no signal derived from the formulation buffer components.

The sample containing formulation buffer + 0.4 mg/mL PS20 HP (polysorbate 20 high purity) resulted in a determined PS20 HP content of 0.393±0.014 mg/mL PS20 HP.

The sample containing 20 mg/mL mAb4 in the formulation buffer + 0.4 mg/mL PS20 HP (polysorbate 20 high purity) resulted in a determined PS20 HP content of 0.404±0.013 mg/mL PS20 HP. The target PS20 HP concentration was 0.4 mg/mL.

### - mAb5 (IgG4)

mAb5 is also an IgG4 format and was presented in the same formulation as mAb4. As polysorbate the PS20 HP (polysorbate 20 high purity) was used.

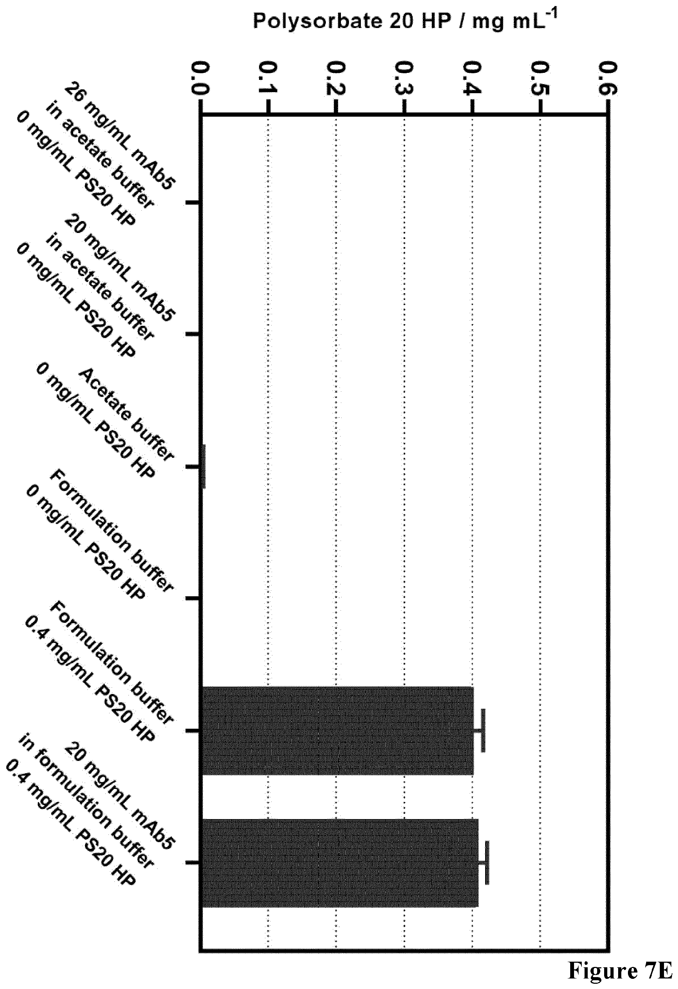

Figure 7E shows the results of the specificity evaluation of the FMA, performed with the following samples:
- 26 mg/mL mAb5 in acetate buffer without polysorbate
- 20 mg/mL mAb5 in acetate buffer without polysorbate,
- pure acetate buffer without polysorbate
- pure formulation buffer without polysorbate
- formulation buffer + 0.4 mg/mL PS20 HP (polysorbate 20 high purity) and
- 20 mg/mL mAb5 in the formulation buffer + 0.4 mg/mL PS20 HP (polysorbate 20 high purity).

The FMA results of the specificity testing showed no signal for the acetate Ultrafiltration/Diafiltration (UF/DF) buffer and mAb5 at concentrations of 26 mg/mL and 20 mg/mL, respectively, in acetate buffer for the polysorbate-free samples. There was also no signal resulting from the formulation buffer without added PS20 HP.

The sample containing formulation buffer + 0.4 mg/mL PS20 HP (polysorbate 20 high purity) resulted in a determined PS20 HP content of 0.402±0.014 mg/mL PS20 HP.

The sample containing 20 mg/mL mAb5 in the formulation buffer + 0.4 mg/mL PS20 HP (polysorbate 20 high purity) resulted in a determined PS20 HP content of 0.409±0.013 mg/mL PS20 HP. The target PS20 HP concentration was 0.4 mg/mL.

### - alpha-lactalbumin

The standard protein alpha-lactalbumin was also investigated for the specificity in the FMA. Due to the low solubility, a concentration of 0.5 mg/mL was selected. As polysorbate the PS20 HP (polysorbate 20 high purity) was used.

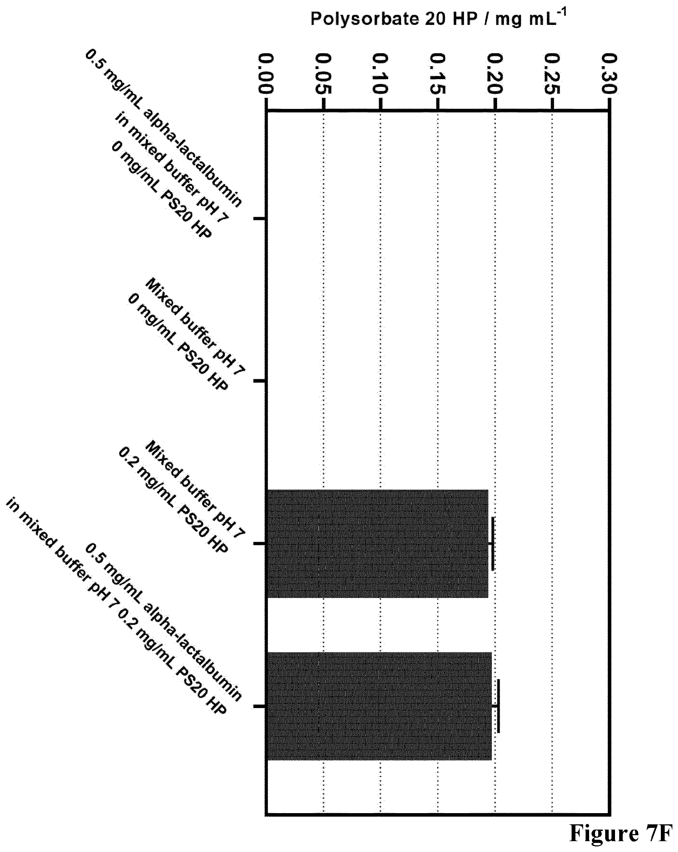

Figure 7F shows the results of the specificity evaluation of the FMA, performed with the following samples:
- 0.5 mg/mL alpha-lactalbumin in mixed buffer, pH 7, without polysorbate
- pure mixed buffer, pH 7, without polysorbate
- mixed buffer, pH 7, + 0.2 mg/mL PS20 HP (polysorbate 20 high purity) and
- 0.5 mg/mL alpha-lactalbumin in mixed buffer, pH 7, + 0.2 mg/mL PS20 HP (polysorbate 20 high purity).

Alpha-lactalbumin and the buffer system in the polysorbate-free samples did not show a signal in the FMA. The FMA of the sample containing mixed buffer with added 0.2 mg/mL PS20 HP resulted in 0.194±0.004 mg/mL PS20 HP and the sample containing the protein dissolved at a concentration of 0.5 mg/mL in this buffer with added 0.2 mg/mL PS20 HP resulted in 0.197±0.006 mg/mL PS20 HP. The target PS20 HP concentration was 0.2 mg/mL

### 2.2.3 Accuracy

The accuracy of an analytical procedure expresses the closeness of agreement between the value, which is accepted as either a conventional true value or an accepted reference value and the value found [6]. Accuracy was first evaluated for the FMA by spiking known amounts of different PS20 HP concentrations in acetate buffer. The investigated PS20 HP spike concentrations were 0.1, 0.3, and 0.6 mg/mL PS20 HP. The spike recovery rate was calculated according to equation I: $Spike recovery rate = \left(\frac{result of analysis}{theoretical spike value}\right) \times 100$

The target PS20 HP concentration was 0.4 mg/mL PS20 HP. The accuracy was tested at 25 %, 75 %, and 150 % of the target PS20 HP concentration. The results of the accuracy testing are summarized in the following Table 2:

**Table 2: Results of accuracy testing (n=12) of PS20 HP in acetate buffer**

| Spike | Spiked PS20 HP [mg/mL] | Protein concentration [mg/mL] | Determined PS20 HP by FMA (n=12) [mg/mL] | Spike recovery rate PS20 HP (n=12) [%] |
|---|---|---|---|---|
| 0.1 mg/ml PS20 HP | 0.1 | 0 | 0.08 - 0.104 | 80 - 104 |
| 0.3 mg/ml PS20 HP | 0.3 | 0 | 0.297 - 0.351 | 99 - 117 |
| 0.6 mg/ml PS20 HP | 0.6 | 0 | 0.582 - 0.648 | 97 - 108 |

As may be seen from Table 2, for the samples containing PS20 HP in acetate buffer, the spike recovery rates were in a range of 80 - 104 % for samples containing 25 % of the target PS20 HP content, 99 - 117 % for samples containing 75 % of the PS20 HP target, and 97 - 108 % at 150 % of the target PS20 HP concentration, respectively.

### - mAb1 (IgG1)

In a next step, the accuracy of the FMA was assessed in the mAb1 formulations, where the target PS20 HP concentration was 0.4 mg/mL PS20 HP. Accuracy was tested at 25 %, 75 %, and 150 % of the target PS20 HP concentration. The results of the accuracy testing are summarized in Table 3:

**Table 3: Results of accuracy testing (n=12) of PS20 HP in a 150 mg/mL mAb1 solution in acetate buffer**

| Spike | Spiked PS20 HP [mg/mL] | mAb1 concentration [mg/mL] | Determined PS20 HP by FMA (n=12) [mg/mL] | Spike recovery rate PS20 HP (n=12) [%] |
|---|---|---|---|---|
| 0.1 mg/ml PS20 HP | 0.1 | 150 | 0.073 - 0.094 | 73 - 94 |
| 0.3 mg/ml PS20 HP | 0.3 | 150 | 0.298 - 0.352 | 99 - 117 |

| Spike | Spiked PS20 HP [mg/mL] | mAb1 concentration [mg/mL] | Determined PS20 HP by FMA (n=12) [mg/mL] | Spike recovery rate PS20 HP (n=12) [%] |
|---|---|---|---|---|
| 0.6 mg/ml PS20 HP | 0.6 | 150 | 0.581 - 0.629 | 97 - 105 |

As may be seen from Table 3, for the samples containing PS20 HP and 150 mg/mL mAb1 in acetate buffer, the spike recovery rates were determined to be in a range of 73 - 94 % for samples containing 25 % of the target PS20 HP content, 99 - 117 % for samples containing 75 % of the PS20 HP target, and 97 - 105 % at 150 % of the target PS20 HP concentration, respectively.

### - mAb2 (IgG1)

The accuracy of the FMA was assessed in the mAb2 formulations, where the target PS20 HP concentration was 0.4 mg/mL PS20 HP. Accuracy was tested at 25 %, 50 %, 75 %, and 100 % of the target PS20 HP concentration. The results of the accuracy testing are summarized in Table 4:

**Table 4: Results of accuracy testing (n=12) of PS20 HP in a 65 mg/mL mAb2 solution in a histidine buffer**

| Spike | Spiked PS20 HP [mg/mL] | mAb2 concentration [mg/mL] | Determined PS20 HP by FMA (n=12) [mg/mL] | Spike recovery rate PS20 HP (n=12) [%] |
|---|---|---|---|---|
| 0.1 mg/ml PS20 HP | 0.1 | 65 | 0.083 - 0.110 | 83 - 110 |
| 0.2 mg/ml PS20 HP | 0.2 | 65 | 0.187 - 0.220 | 94 - 110 |
| 0.3 mg/ml PS20 HP | 0.3 | 65 | 0.268 - 0.315 | 89 - 105 |
| 0.4 mg/ml PS20 HP | 0.4 | 65 | 0.368 - 0.424 | 92 -106 |

As may be seen from Table 4, for the samples containing PS20 HP and 65 mg/mL mAb2 in histidine buffer, the spike recovery rates were determined to be in a range of 83 - 110 % for samples containing 25 % of the target PS20 HP content, 94 - 110 % for samples containing 50 % of the target PS20 HP content, 89 - 105 % for samples containing 75 % of the PS20 HP target, and 92 - 106 % at 100 % of the target PS20 HP concentration, respectively.

### - mAb3 (IgG1)

The accuracy of the FMA was assessed in the mAb3 formulations, where the target PS80 HP concentration was 0.5 mg/mL PS80 HP. Accuracy was tested at 20 %, 40 %, 60 %, and 120 % of the target PS80 HP concentration. The results of the accuracy testing are summarized in Table 5:

**Table 5: Results of accuracy testing (n=4) of PS80 HP in 100 mg/mL mAb3 solution in acetate buffer**

| Spike | Spiked PS80 HP [mg/mL] | mAb3 concentration [mg/mL] | Determined PS80 HP by FMA (n=4) [mg/mL] | Spike recovery rate PS80 HP (n=4) [%] |
|---|---|---|---|---|
| 0.1 mg/ml PS80 HP | 0.1 | 100 | 0.079 - 0.111 | 79 - 111 |
| 0.2 mg/ml PS80 HP | 0.2 | 100 | 0.214 - 0.236 | 107 - 118 |
| 0.3 mg/ml PS80 HP | 0.3 | 100 | 0.324 - 0.340 | 108 - 113 |
| 0.6 mg/ml PS80 HP | 0.6 | 100 | 0.483 - 0.587 | 81 - 98 |

As may be seen from Table 5, for the samples containing PS80 HP and 100 mg/mL mAb3 in acetate buffer, the spike recovery rates were determined to be in a range of 79 - 111 % for samples containing 20 % of the target PS80 HP content, 107 - 118 % for samples containing 40 % of the target PS80 HP content, 108 - 113 % for samples containing 60 % of the PS80 HP target, and 81 - 98 % at 120 % of the target PS80 HP concentration, respectively.

### - mAb4 (IgG4)

The accuracy of the FMA was assessed in the mAb4 formulations, where the target PS20 HP concentration was 0.4 mg/mL PS20 HP. Accuracy was tested at 25 %, 75 %, and 150 % of the target PS20 HP concentration. The results of the accuracy testing are summarized in Table 6:

**Table 6: Results of accuracy testing of the FMA for PS20 HP in a 20 mg/mL mAb4 solution in acetate buffer**

| Spike | Spiked PS20 HP [mg/mL] | mAb4 concentration [mg/mL] | Determined PS20 HP by FMA (n=12) [mg/mL] | Spike recovery rate PS20 HP (n=12) [%] |
|---|---|---|---|---|
| 0.1 mg/ml PS20 HP | 0.1 | 20 | 0.085 - 0.106 | 85 - 106 |
| 0.3 mg/ml PS20 HP | 0.3 | 20 | 0.294 - 0.327 | 98 - 109 |
| 0.6 mg/ml PS20 HP | 0.6 | 20 | 0.528 - 0.600 | 88 - 100 |

As may be seen from Table 6, for the samples containing PS20 HP and 20 mg/mL mAb4 in acetate buffer, the spike recovery rates were determined to be in a range of 85 - 106 % for samples containing 25 % of the target PS20 HP content, 98 - 109 % for samples containing 75 % of the PS20 HP target, and 88 - 100 % at 150 % of the target PS20 HP concentration, respectively.

### - mAb5 (IgG4)

The accuracy of the FMA was assessed in the mAb5 formulations, where the target PS20 HP concentration was 0.4 mg/mL PS20 HP. Accuracy was tested at 25 %, 50 %, 100 %, and 150 % of the target PS20 HP concentration. The results of the accuracy testing are summarized in Table 7:

**Table 7: Results of accuracy testing (n=8) of PS20 HP in a 20 mg/mL mAb5 solution in acetate buffer**

| Spike | Spiked PS20 HP [mg/mL] | mAb5 concentration [mg/mL] | Determined PS20 HP by FMA (n=8) [mg/mL] | Spike recovery rate PS20 HP (n=8) [%] |
|---|---|---|---|---|
| 0.1 mg/ml PS20 HP | 0.1 | 20 | 0.095 - 0.115 | 95 - 115 |
| 0.2 mg/ml PS20 HP | 0.2 | 20 | 0.196 - 0.227 | 98 - 114 |
| 0.4 mg/ml PS20 HP | 0.4 | 20 | 0.410 - 0.436 | 103 - 109 |
| 0.6 mg/ml PS20 HP | 0.6 | 20 | 0.553 - 0.613 | 92 - 102 |

As may be seen from Table 7, for the samples containing PS20 HP and 20 mg/mL mAb5 in acetate buffer, the spike recovery rates were determined to be in a range of 95 - 115 % for samples containing 25 % of the target PS20 HP content, 98 - 114 % for samples containing 50 % of the target PS20 HP content, 103 - 109 % for samples containing 100 % of the PS20 HP target, and 92 - 102 % at 150 % of the target PS20 HP concentration, respectively.

### -Lysozyme

The accuracy of the FMA was assessed in the lysozyme formulations, where the target PS80 HP concentration was 0.2 mg/mL PS80 HP. Accuracy was tested at 50 %, 100 %, and 150 % of the target PS80 HP concentration. Lysozyme is a protein and an enzyme which consists of 129 amino acids. The results of the accuracy testing are summarized in Table 8:

**Table 8: Results of accuracy testing (n=8) of PS80 HP in a 10 mg/mL lysozyme solution in phosphate buffered saline (PBS)**

| Spike | Spiked PS80 HP [mg/mL] | lysozyme concentration [mg/mL] | Determined PS80 HP by FMA (n=16) [mg/mL] | Spike recovery rate PS80 HP (n=16) [%] |
|---|---|---|---|---|
| 0.1 mg/ml PS80 HP | 0.1 | 10 | 0.086 - 0.103 | 86 - 103 |
| 0.2 mg/ml PS80 HP | 0.2 | 10 | 0.184 - 0.204 | 92 - 102 |
| 0.3 mg/ml PS80 HP | 0.3 | 10 | 0.254 - 0.294 | 85 - 98 |

As may be seen from Table 8, for the samples containing PS80 HP and 10 mg/mL lysozyme in PBS buffer, the spike recovery rates were determined to be in a range of 86 - 103 % for samples containing 50 % of the target PS80 HP content, 92 - 102 % for samples containing 100 % of the target PS80 HP content, and 85 - 98 % at 150 % of the target PS80 HP concentration, respectively.

### 2.2.4 Precision

The precision of an analytical procedure expresses the closeness of agreement (degree of scatter) between a series of measurements obtained from multiple sampling of the same homogeneous sample under the prescribed conditions [6]. For the FMA performed on a liquid-handling station, the precision was tested for all mAbs under the respective formulation conditions containing the target PS HP concentration. Every sample was analyzed multiple times, each as a 4x technical replicate. The results for mAb1 are listed in Table 8. For this protein, the %CV (coefficient of variation, also known as relative standard deviation) of each quadruplicate was 3.6 % at maximum, the intra-assay variability (average %CV) was determined to be 2.2 % for mAb1.

**Table 8: Results of precision testing for eight replicates (each n=4) for 150 mg/mL mAb1 in formulation buffer + 0.4 mg/mL PS20 HP. The maximum %CV of each quadruplicate was 3.6 %.**

| Experiment | CV |
|---|---|
| | [%] |
| 1 | 3.6 |
| 2 | 2.7 |
| 3 | 0.6 |
| 4 | 1.6 |
| 5 | 3.0 |
| 6 | 1.7 |
| 7 | 2.5 |
| 8 | 2.0 |

The precision was also investigated for the other mAbs of this study. The results showed an average %CV of 2.5 % (maximum 3.5 %, six replicates, each n=4) for mAb2, 3.6 % (maximum 6.8 %, eight replicates, each n=4) for mAb3, 2.6 % (maximum 3.5 %, six replicates, each n=4) for mAb4, and 2.2 % (maximum 5.0 %, eight replicates, each n=4) for mAb5. All results are summarized in Table 9:

**Table 9: Summary of the evaluation of precision for the FMA conducted with mAb1 - mAb5.**

| Protein | Protein concentration [mg/mL] | PS type | PS concentration [mg/mL] | Replicates [-] | Average %CV all technical replicates [%] | Maximum %CV among all technical replicates [%] |
|---|---|---|---|---|---|---|
| mAb1 | 150 | PS20 HP | 0.4 | 8x 4 | 2.2 | 3.6 |
| mAb2 | 65 | PS20 HP | 0.2 | 6x 4 | 2.5 | 3.5 |
| mAb3 | 100 | PS80 HP | 0.5 | 8x 4 | 3.6 | 6.8 |
| mAb4 | 20 | PS20 HP | 0.4 | 6x 4 | 2.6 | 3.5 |
| mAb5 | 20 | PS20 HP | 0.4 | 8x 4 | 2.2 | 5.0 |

### 2.2.5 Determination of LOD and LOQ

For the FMA conducted in microbatch format, the Limit of Detection (LOD) and the Limit of Quantitation (LOQ) were determined according to [14]. Determined values for PS20 HP were 0.023 mg/mL LOD and 0.069 mg/mL LOQ. In contrast to PS20 HP, for PS20 China grade, the LOD was 0.009 mg/mL and the LOQ was 0.028 mg/mL. For PS80 HP, the determined values were 0.001 mg/mL for the LOD and 0.003 mg/mL for the LOQ. The LODs and LOQs of PS20 HP, PS20 China grade and PS80 HP are listed in the following Table 10.

**Table 10: Results of the LOD / LOQ testing of PS20 HP, PS20 China grade and PS80 HP.**

| | LOD | LOQ |
|---|---|---|
| | [mg/mL] | [mg/mL] |
| PS20 HP | 0.023 | 0.069 |
| PS20 China grade | 0.009 | 0.028 |
| PS80 HP | 0.001 | 0.003 |

### List of reference signs

- 100a-f, 187: sample
- 125: solvent
- 130, 130.1, 130.2, 130.3: polysorbate molecule
- 150.1, 350.1: irradiated light of 350 nm
- 150.2, 350.2: emitted light of 420 nm
- 165, 195: fluorescence detector
- 175: degraded polysorbate
- 110a, 110b, 110c: polysorbate micelles
- 120a, 120b: not intact micelles
- 180: mobile phase
- 182, 188: capillary tubing
- 185: pump
- 190: reaction loop
- 197: waste
- 210.1, 210.2, 210.3, 210.4: sample
- 220a, 220b, 220c: polysorbate micelles
- 310.1, 310.2, 310.3, 310.4: feeding system
- 300: automated system or device
- 400: microplate
- NPN: N-phenyl-1-naphthylamine

### References

**1.** Jones MT, Mahler H-C, Yadav S, Bindra D, Corvari V, Fesinmeyer RM, et al. Considerations for the Use of Polysorbates in Biopharmaceuticals. Pharmaceut Res. 2018;35:148.
**2.** Dwivedi M, Blech M, Presser I, Garidel P. Polysorbate degradation in biotherapeutic formulations: Identification and discussion of current root causes. Int J Pharmaceut. 2018;552:422-36.
**3.** Lippold S, Koshari SHS, Kopf R, Schuller R, Buckel T, Zarraga IE, et al. Impact of mono- and polyester fractions on polysorbate quantitation using mixed-mode HPLC-CAD/ELSD and the fluorescence micelle assay. J Pharmaceut Biomed. 2017;132:24-34.
**4.** Garidel P, Blech M, Buske J, Blume A. Surface Tension and Self-association Properties of Aqueous Polysorbate 20 HP and 80 HP Solutions: Insights into Protein Stabilisation Mechanisms. J Pharm Innov. 2020;1-9.
**5.** Brito RMM, Vaz WLC. Determination of the critical micelle concentration of surfactants using the fluorescent probe N-phenyl-1-naphthylamine. Anal Biochem. 1986;152:250-5.
**6.** Mahler H, Printz M, Kopf R, Schuller R, Miller R. Behaviour of polysorbate 20 during dialysis, concentration and filtration using membrane separation techniques. J Pharm Sci. 2008;97:764-74.
**7.** Wuelfing WP, Kosuda K, Templeton AC, Harman A, Mowery MD, Reed RA. Polysorbate 80 UV/vis spectral and chromatographic characteristics-definingboundary conditions for use of the surfactant in dissolution analysis. J PharmBiomed Anal. 2006;41(3):774-782.
**8.** Khossravi M, Kao Y-H, Mrsny RJ, Sweeney TD. Analysis Methods of Polysorbate 20: A New Method to Assess the Stability of Polysorbate 20 and Established Methods That May Overlook Degraded Polysorbate 20. Pharmaceut Res. 2002;19:634-9.
**9.** Martos A, Berger M, Kranz W, Spanopoulou A, Menzen T, Friess W, et al. Novel High-Throughput Assay for Polysorbate Quantification in Biopharmaceutical Products by Using the Fluorescent Dye Dil. J Pharm Sci. 2020;109:646-55.
**10.** Martos A, Koch W, Jiskoot W, Wuchner K, Winter G, Friess W, et al. Trends on Analytical Characterization of Polysorbates and Their Degradation Products in Biopharmaceutical Formulations. J Pharm Sci. 2017;106:1722-35.
**11.** Kishore RS, Pappenberger A, Dauphin IB, et al. Degradation of polysorbates 20 and 80: studies on thermal autoxidation and hydrolysis. J Pharm Sci. 2011;100(2):721-731.
**12.** Kishore RSK, Kiese S, Fischer S, Pappenberger A, Grauschopf U, Mahler H-C. The Degradation of Polysorbates 20 and 80 and its Potential Impact on the Stability of Biotherapeutics. Pharmaceut Res. 2011;28:1194-1210.
**13.** Evers D-H, Carle S, Lakatos D, Hämmerling F, Garidel P, Buske J. Hydrolytic polysorbate 20 degradation - sensitive detection of free fatty acids in biopharmaceuticals via UPLC-QDa analytics with isolator column. J Chromatogr B. 2021;122717.
**14.** ICH International Conference on Harmonisation of Technical Requirements for Registration of Pharmaceuticals for Human Use: ICH Harmonised Tripartite Guideline; Validation of Analytical Procedures: Text and Methodology Q2(R1). pages 1 - 13. 2005.
**15.** Kromidas, Stavros, and Kuss, Hans -Joachim. Handbuch Validierung in der Analytik. Weinheim: John Wiley & Sons, Incorporated, 2009.pages: 360 - 366.
**16.** Fekete S, Ganzler K, Fekete J. Fast and sensitive determination of Polysorbate 80 in solutions containing proteins. J Pharmaceut Biomed. 2010;52:672-9.
**17.** Bhambure R, Kumar K, Rathore AS. High-throughput process development for biopharmaceutical drug substances. Trends Biotechnol. 2011;29:127-35.

## Claims

1. A method for the quantification of polysorbate in an aqueous formulation containing polysorbate and one or more polypeptides having the following steps:
(i) providing a number of obtained samples of the aqueous formulation;
(ii) adding of a solution containing the fluorescent dye N-phenyl-1-naphthylamine to each of the samples, respectively, to obtain fluorescent dye containing samples to be tested in a fluorescence micelle assay;
(iii) performing a fluorescence micelle assay with the fluorescent dye containing samples obtained in step (ii); and
(iv) determining the content of polysorbate present in each of the samples from the fluorescence micelle assay performed in step (iii);
with the proviso that the method is being performed without the use of a HPLC (high performance liquid chromatrography)-system,
**characterized in that**
the method steps (i) to (iv) are performed on a microplate.

2. The method according to claim 1, wherein the method comprises a step (ii') of incubating the fluorescent dye containing samples of step (ii), wherein step (ii') is performed after step (ii) and before step (iii), and wherein step (iii) is performed with the fluorescent dye containing samples obtained in step (ii').

3. The method according to claim 1 or 2,
**characterized in that**
one, two, three or more of the following conditions are fulfilled:
- the incubating in step (ii') is performed under shaking;
- the samples of the aqueous formulation in step (i) are provided at the same time or one after the other;
- the solution containing the fluorescent dye N-phenyl-1-naphthylamine in step (ii) is added to each of the samples at the same time or one after the other;
- the incubating in step (ii') is realized for the samples at the same time or one after the other;
- the incubating in step (ii') is performed under shaking and realized for the samples at the same time or one after the other;
- the incubating in step (ii') is performed at a temperature selected from the range of from 10 to 60 °C, particularly 30 to 40 °C; more particularly 35 °C; and/or
- the method is performed with the use of a fluorescence detector.

4. The method according to any of the preceding claims 1 to 3,
**characterized in that**
mixing the solution containing the fluorescent dye N-phenyl-1-naphthylamine to remove any concentration gradient of the dye, prior to adding it in step (ii), whereby the mixing is conducted by shaking or stirring.

5. The method according to any of the preceding claims 1 to 4,
**characterized in that**
the solution containing the fluorescent dye in step (ii) has the same components as used for a mobile phase in a combined FMA-HPLC method to quantify polysorbate, particularly contains the hydrophobic dye in the range from 1 µM to 10 µM, particularly 5 µM, an organic solvent in the range from 1 %(v/v) to 10 %(v/v), particularly 5 %(v/v), a buffer in the range from 5 mM to 500 mM, particularly 50 mM, a salt in the range from 10 mM to 1000 mM, particularly 150 mM, and a tenside, especially a non-ionic tenside, in the range from 0.00015 % to 0.015 %, particularly 0.0015 %.

6. The method according to any of the preceding claims 1 to 5,
**characterized in that**
the polysorbate is
- intended for pharmaceutical use, particularly for use in drugs; and/or
- selected from polysorbate 20, polysorbate 40, polysorbate 60, or polysorbate 80, particularly polysorbate 20 or polysorbate 80; and/or
- selected from polysorbate 20 or polysorbate 80, which are suitable for parenteral administration.

7. The method according to any of the preceding claims 1 to 6,
**characterized in that**
the method is performed using disposable labware having non-binding, low-binding or medium-binding surfaces, particularly non-binding surface.

8. The method according to any of the preceding claims 1 to 7,
**characterized in that**
a part or all of the method steps (i) to (iv) are automated.

9. The method according to any of the preceding claims 1 to 8,
**characterized in that**
the polypeptide(s) is(are) selected from a monoclonal antibody, polyclonal antibody, monospecific antibody, multispecific antibody, single chain antibodies or antibody fragments thereof, such as, Fv, scFv, Fab, Fab', scFab, F(ab')2, Fab2, Fc and Fc'-fragments, heavy and light immunoglobulin chains and their constant, variable or hypervariable region as well as Fv- and Fd-fragments, diabody, triabody, scFv-Fc, minibody, or single domain antibody as well as one or more enzymes or a mixture thereof.

10. The method according to any of the preceding claims 1 to 9,
**characterized in that**
the polypeptide(s) is(are) selected from therapeutic polypeptide(s), which are used for the prevention or treatment of a disease or a disorder.

11. The method according to any of the preceding claims 1 to 10,
**characterized in that**
a biopharmaceutic formulation is used as the aqueous formulation.

12. The method according to any of the preceding claims 1 to 11,
wherein the method is used during the development, manufacture, shelf life, or storage time thereof, whereby the aqueous formulation contains polysorbate and one or more polypeptides.

## Patentansprüche

1. Verfahren zur Quantifizierung von Polysorbat in einer wässrigen Formulierung, enthaltend Polysorbat und ein oder mehrere Polypeptide, mit den nachfolgenden Schritten:
(i) Bereitstellen einer Anzahl von erhaltenen Proben der wässrigen Formulierung;
(ii) Zugeben einer Lösung, enthaltend den Fluoreszenzfarbstoff N-Phenyl-1-naphthylamin, zu jeder der Proben, um fluoreszenzfarbstoffhaltige Proben zu erhalten, die in einem Fluoreszenzmizellen-Assay getestet werden sollen;
(iii) Durchführen eines Fluoreszenzmizellen-Assays mit den in Schritt (ii) erhaltenen fluoreszenzfarbstoffhaltigen Proben; und
(iv) Bestimmen des Gehalts an Polysorbat, das in jeder der Proben vorhanden ist, anhand des in Schritt (iii) durchgeführten Fluoreszenzmizellen-Assays;
mit der Maßgabe, dass das Verfahren ohne Verwendung eines HPLC-(Hochleistungsflüssigkeitschromatographie)-Systems durchgeführt wird,
**dadurch gekennzeichnet, dass**
die Verfahrensschritte (i) bis (iv) auf einer Mikrotiterplatte durchgeführt werden.

2. Verfahren nach Anspruch 1, wobei das Verfahren einen Schritt (ii') umfasst, bei dem die fluoreszenzfarbstoffhaltigen Proben aus Schritt (ii) inkubiert werden, wobei Schritt (ii') nach Schritt (ii) und vor Schritt (iii) durchgeführt wird und wobei Schritt (iii) mit den in Schritt (ii') erhaltenen, fluoreszenzfarbstoffhaltigen Proben durchgeführt wird.

3. Verfahren nach Anspruch 1 oder 2,
**dadurch gekennzeichnet, dass**
eine, zwei, drei oder mehr der folgenden Bedingungen erfüllt sind:
- die Inkubation in Schritt (ii') wird unter Schütteln durchgeführt;
- die Proben der wässrigen Formulierung werden in Schritt (i) gleichzeitig oder nacheinander bereitgestellt;
- die Lösung, die den Fluoreszenzfarbstoff N-Phenyl-1-naphthylamin enthält, wird in Schritt (ii) jeder der Proben gleichzeitig oder nacheinander zugegeben;
- die Inkubation in Schritt (ii') wird für die Proben gleichzeitig oder nacheinander durchgeführt;
- die Inkubation in Schritt (ii') wird unter Schütteln durchgeführt und wird für die Proben gleichzeitig oder nacheinander durchgeführt;
- die Inkubation in Schritt (ii') wird bei einer Temperatur durchgeführt, ausgewählt aus dem Bereich von 10 bis 60 °C, insbesondere 30 bis 40 °C; insbesondere bei 35 °C; und/oder
- das Verfahren wird unter Verwendung eines Fluoreszenzdetektors durchgeführt.

4. Verfahren nach irgendeinem der vorstehenden Ansprüche 1 bis 3,
**dadurch gekennzeichnet, dass**
die Lösung, enthaltend den Fluoreszenzfarbstoff N-Phenyl-1-naphthylamin, vor ihrer Zugabe in Schritt (ii) gemischt wird, um einen etwaigen Konzentrationsgradienten des Farbstoffs zu beseitigen, wobei das Mischen durch Schütteln oder Rühren erfolgt.

5. Verfahren nach irgendeinem der vorstehenden Ansprüche 1 bis 4,
**dadurch gekennzeichnet, dass**
die den Fluoreszenzfarbstoff enthaltende Lösung in Schritt (ii) dieselben Bestandteile aufweist, wie sie für eine mobile Phase in einem kombinierten FMA-HPLC-Verfahren zur Quantifizierung von Polysorbat verwendet werden, insbesondere den hydrophoben Farbstoff im Bereich von 1 µM bis 10 µM, insbesondere 5 µM, ein organisches Lösungsmittel im Bereich von 1 %(v/v) bis 10 %(v/v), insbesondere 5 %(v/v), einen Puffer im Bereich von 5 mM bis 500 mM, insbesondere 50 mM, ein Salz im Bereich von 10 mM bis 1000 mM, insbesondere 150 mM, und ein Tensid, insbesondere ein nichtionisches Tensid, im Bereich von 0,00015 % bis 0,015 %, insbesondere 0,0015 %, enthält.

6. Verfahren nach irgendeinem der vorstehenden Ansprüche 1 bis 5,
**dadurch gekennzeichnet, dass**
das Polysorbat
- für pharmazeutische Zwecke, insbesondere zur Verwendung in Arzneimitteln, bestimmt ist; und/oder
- aus Polysorbat 20, Polysorbat 40, Polysorbat 60 oder Polysorbat 80, insbesondere Polysorbat 20 oder Polysorbat 80, ausgewählt ist; und/oder
- aus Polysorbat 20 oder Polysorbat 80 ausgewählt ist, die für die parenterale Verabreichung geeignet sind.

7. Verfahren nach irgendeinem der vorstehenden Ansprüche 1 bis 6,
**dadurch gekennzeichnet, dass**
das Verfahren unter Verwendung von Einweg-Laborgeräten mit nicht bindenden, schwach bindenden oder mäßig bindenden Oberflächen, insbesondere mit nicht bindenden Oberflächen, durchgeführt wird.

8. Verfahren nach irgendeinem der vorstehenden Ansprüche 1 bis 7,
**dadurch gekennzeichnet, dass**
ein Teil der oder alle Verfahrensschritte (i) bis (iv) automatisiert sind.

9. Verfahren nach irgendeinem der vorstehenden Ansprüche 1 bis 8,
**dadurch gekennzeichnet, dass**
das Polypeptid bzw. die Polypeptide ausgewählt sind aus einem monoklonalen Antikörper, einem polyklonalen Antikörper, einem monospezifischen Antikörper, einem multispezifischen Antikörper, Einzelketten-Antikörpern oder Antikörperfragmenten davon, wie beispielsweise Fv, scFv, Fab, Fab', scFab, F(ab')2, Fab2, Fc- und Fc'-Fragmenten, schweren und leichten Immunglobulinketten und deren konstante, variable oder hypervariable Region sowie Fv- und Fd-Fragmente, Diabody, Triabody, scFv-Fc, Minibody oder Single-Domain-Antikörper sowie einem oder mehreren Enzymen oder eine Mischung davon.

10. Verfahren nach irgendeinem der vorstehenden Ansprüche 1 bis 9,
**dadurch gekennzeichnet, dass**
das Polypeptid bzw. die Polypeptide aus therapeutischen Polypeptiden ausgewählt ist bzw. sind, die zur Vorbeugung oder Behandlung einer Krankheit oder einer Störung eingesetzt werden.

11. Verfahren nach irgendeinem der vorstehenden Ansprüche 1 bis 10,
**dadurch gekennzeichnet, dass**
eine biopharmazeutische Formulierung als die wässrige Formulierung verwendet wird.

12. Verfahren nach irgendeinem der vorstehenden Ansprüche 1 bis 11,
wobei das Verfahren während der Entwicklung, Herstellung, Haltbarkeit oder Lagerzeit desselben angewendet wird, wobei die wässrige Formulierung Polysorbat und ein oder mehrere Polypeptide enthält.

## Revendications

1. Méthode de quantification du polysorbate dans une formulation aqueuse contenant du polysorbate et un ou plusieurs polypeptides présentant les étapes suivantes :
(i) la fourniture d'un certain nombre d'échantillons obtenus de la formulation aqueuse ;
(ii) l'ajout d'une solution contenant le colorant fluorescent N-phényl-1-naphthylamine à chacun des échantillons, respectivement, pour obtenir des échantillons contenant le colorant fluorescent à tester dans un dosage de micelles par fluorescence ;
(iii) la réalisation d'un dosage de micelles par fluorescence avec les échantillons contenant le colorant fluorescent obtenus à l'étape (ii) ; et
(iv) la détermination de la teneur en polysorbate présent dans chacun des échantillons à partir du dosage de micelles par fluorescence réalisé à l'étape (iii) ;
à condition que la méthode soit réalisée sans l'utilisation d'un système HPLC (chromatographie en phase liquide à haute performance),
**caractérisée en ce que**
les étapes de méthode (i) à (iv) sont réalisées sur une microplaque.

2. Méthode selon la revendication 1, dans laquelle la méthode comprend une étape (ii') d'incubation des échantillons contenant le colorant fluorescent de l'étape (ii), dans laquelle l'étape (ii') est réalisée après l'étape (ii) et avant l'étape (iii), et dans laquelle l'étape (iii) est réalisée avec les échantillons contenant le colorant fluorescent obtenus à l'étape (ii').

3. Méthode selon la revendication 1 ou 2,
**caractérisée en ce que**
une, deux, trois ou plus des conditions suivantes sont satisfaites :
- l'incubation à l'étape (ii') est réalisée sous secouage ;
- les échantillons de la formulation aqueuse à l'étape (i) sont fournis simultanément ou l'un après l'autre ;
- la solution contenant le colorant fluorescent N-phényl-1-naphthylamine à l'étape (ii) est ajoutée à chacun des échantillons simultanément ou l'un après l'autre ;
- l'incubation à l'étape (ii') est réalisée pour les échantillons simultanément ou l'un après l'autre ;
- l'incubation à l'étape (ii') est réalisée sous secouage et exécutée pour les échantillons simultanément ou l'un après l'autre ;
- l'incubation à l'étape (ii') est réalisée à une température sélectionnée dans la plage de 10 à 60 °C, en particulier de 30 à 40 °C ; plus particulièrement 35 °C ; et/ou
- la méthode est réalisée à l'aide d'un détecteur de fluorescence.

4. Méthode selon l'une quelconque des revendications 1 à 3 précédentes,
**caractérisée par**
le mélange de la solution contenant le colorant fluorescent N-phényl-1-naphthylamine pour éliminer tout gradient de concentration du colorant, avant de l'ajouter à l'étape (ii), selon laquelle le mélange est effectué par secouage ou agitation.

5. Méthode selon l'une quelconque des revendications 1 à 4 précédentes,
**caractérisée en ce que**
la solution contenant le colorant fluorescent à l'étape (ii) présente les mêmes composants que ceux utilisés pour une phase mobile dans une méthode combinée FMA-HPLC pour quantifier le polysorbate, contient en particulier le colorant hydrophobe dans la plage de 1 µM à 10 µM, en particulier 5 µM, un solvant organique dans la plage de 1 % (v/v) à 10 % (v/v), en particulier 5 %(v/v), un tampon dans la plage de 5 mM à 500 mM, en particulier 50 mM, un sel dans la plage de 10 mM à 1 000 mM, en particulier 150 mM, et un tensioactif, notamment un tensioactif non ionique, dans la plage de 0,00015 % à 0,015 %, en particulier 0,0015 %.

6. Méthode selon l'une quelconque des revendications 1 à 5 précédentes,
**caractérisée en ce que**
le polysorbate est
- destiné à un usage pharmaceutique, en particulier pour son utilisation dans des médicaments ; et/ou
- sélectionné parmi le polysorbate 20, le polysorbate 40, le polysorbate 60 ou le polysorbate 80, en particulier le polysorbate 20 ou le polysorbate 80 ; et/ou
- sélectionné parmi le polysorbate 20 ou le polysorbate 80, qui conviennent à une administration parentérale.

7. Méthode selon l'une quelconque des revendications 1 à 6 précédentes,
**caractérisée en ce que**
la méthode est réalisée à l'aide d'ustensiles de laboratoire jetables présentant des surfaces non liantes, faiblement liantes ou moyennement liantes, en particulier une surface non liante.

8. Méthode selon l'une quelconque des revendications 1 à 7 précédentes,
**caractérisée en ce que**
une partie ou la totalité des étapes de méthode (i) à (iv) sont automatisées.

9. Méthode selon l'une quelconque des revendications 1 à 8 précédentes,
**caractérisée en ce que**
le ou les polypeptides sont sélectionnés parmi un anticorps monoclonal, un anticorps polyclonal, un anticorps monospécifique, un anticorps multispécifique, des anticorps à chaîne unique ou des fragments d'anticorps de ceux-ci, tels que les fragments Fv, scFv, Fab, Fab', scFab, F(ab')2, Fab2, Fc et Fc', des chaînes lourdes et légères d'immunoglobulines et leur région constante, variable ou hypervariable, ainsi que les fragments Fv et Fd, un dianticorps, un trianticorps, un scFv-Fc, un minianticorps, ou un anticorps à domaine unique, ainsi qu'une ou plusieurs enzymes ou un mélange de ceux-ci.

10. Méthode selon l'une quelconque des revendications 1 à 9 précédentes,
**caractérisée en ce que**
le ou les polypeptides sont sélectionnés parmi un ou des polypeptides thérapeutiques, lesquels sont utilisés pour la prévention ou le traitement d'une maladie ou d'un trouble.

11. Méthode selon l'une quelconque des revendications 1 à 10 précédentes,
**caractérisée en ce que**
une formulation biopharmaceutique est utilisée comme formulation aqueuse.

12. Méthode selon l'une quelconque des revendications 1 à 11 précédentes,
dans laquelle la méthode est utilisée pendant le développement, la fabrication, la durée de conservation ou la durée de stockage de celle-ci, selon laquelle la formulation aqueuse contient du polysorbate et un ou plusieurs polypeptides.
